# EUROPEAN PATENT APPLICATION

(11) **EP 2 070 509 A2**
(43) Date of publication of application: **17.06.2009**
(21) Application number: 09156220.7
(22) Date of filing: 05.01.2005
(51) Int. Cl.: A61K 8/04, A61K 8/92, A61Q 19/08

(54) **The use of Myrica gale oil**

(30) Priority: 11.11.2004 GB 0424891
(62) Divisional of application: 05701787.3
(71) Applicant: The Boots Company PLC, Nottingham, Nottinghamshire NG2 3AA (GB)
(72) Inventor: Galley, Edward, Nottingham, NG2 3AA (GB); Smith, Christopher, Nottingham, Nottinghamshire NG16 2UB (GB); Benest, Eilidh, Nottingham, Nottinghamshire NG1 1GF (GB)
(74) Representative: Benest, Eilidh Ruth

(57) **Abstract**

The use of *Myrica gale* oil in the manufacture of a composition for the treatment of the skin or other exposed surfaces of the body is disclosed. *Myrica gale* oil may be used with other active agents which have complementary benefits, or which work with *Myrica gale* oil to produce a particularly efficacious product.

## Description

This invention relates to topical compositions and in particular to cosmetic and/or toiletry compositions for application to the skin and hair.

*Myrica gale* is a robust deciduous shrub that thrives in the wet, acidic soils of the Highlands and Islands of Scotland, particularly in areas that are not easily accessed, farmed or cultivated, eg on steep inclines and in bog areas. The plant is also found in similar habitats in Northern Ireland, northern Europe, Siberia, Canada and northern America. Its success on such infertile land is due partly to its symbiotic association with a nitrogen-fixing fungus which, together with organic matter from its leaves, adds nutrients to the soil. *Myrica gale* produces a range of morphological forms with some types producing larger leaves, and various differences in branching habit and distribution of male, female and/or bisexual flowers. The plant has a unique and pleasant fragrance produced by essential oil in its leaves and catkins.

*Myrica gale* has a number of alternative names, including: Sweet Gale, Bog Myrtle, English Bog, Gale, Gall, Gall Busses, Miortal, Myrtle, Rideag, Roid, Scotch Gale, Highland Gale, Murica Gale, Dutch Myrtle, Herba Myrti Rabantini, Gale Palustris, Flea Wood, Meadow Fern and Tamarix.

As part of mediaeval and traditional herb lore, *Myrica gale* has a long history of use over a wide range of areas. For example, infusions have been consumed to treat stomach and gastric disorders, or used in the form of a wash to treat skin conditions, eg boils and pimples. In European traditions the plant was used as a treatment for scabies, eczema and lice. *Myrica gale* has also been used to flavour food and drink, for example, meats and broths, spirits and beer. Its traditional use as a dye is well documented, and candles have been made from the wax which covers the fruit and leaves.

*Myrica gale* is also an effective insect repellent, and sprigs of the plant can be used to keep away midges, flies and moths, or the leaves can be rubbed onto the skin to release the fragrant oil and keep insects away. GB-A-2224934 discloses an insect repellent gel containing oils obtained by the steam distillation of *Myrica gale* leaves.

*Myrica gale* oil is also known to have use in perfumery, but use of the neat oil is not documented.

The volatile oil consists of a range of monoterpenes and sesquiterpenes (between 120 and 180 components), with approximately 15 compounds having concentrations greater than 1% by weight and constituting about 70% of the total oil. The composition of the oil can vary significantly, although current research shows no direct correlation with different morphological forms of the plant, environmental conditions or even the geographical area of harvest.

A wide variety of topical compositions are used on the skin, hair, or lips for different purposes. Many consumers prefer products that incorporate natural active agents to provide a particular benefit, and products that are pleasant to use and visually attractive.

Common complaints, against which particular benefit is sought, include the signs of ageing, eg fine lines and wrinkles in the skin, and skin disorders eg acne.

The changes associated with ageing, such as formation of lines and wrinkling, actinic lentigines, dyspigmentation, rough skin, actinic telangiectasia and further loss of skin elastic function may be due to direct UV-mediated damage to cells and indirectly mediated damage caused by the generation of free radicals in cells and tissues. This is generally termed photoageing and can account for up to 90% of the skin changes we associate with ageing.

To prevent sunlight-mediated damage to skin cells and associated damage due to sunlight initiating the formation of free radicals in the skin, compositions containing a sunscreen may be used. These compositions generally contain an inorganic sunscreen such as titanium dioxide which reflects the sun's rays, or one or more organic sunscreens which absorb the rays. A further measure to protect the skin is the use of compositions containing antioxidants, which act as free radical quenchers. These react with free radicals and so terminate the chain of reactions that free radicals customarily propagate and which so damage the skin.

Compositions containing sunscreens are common, and some sunscreen formulations also contain antioxidants. There are also cosmetic compositions, not containing sunscreens, which contain antioxidants for additional skin care and protection.

There are a number of skincare compositions, commercially available, which seek to minimise the damage to the skin by the inclusion of antioxidant agents. In particular, materials such as vitamins and herbal extracts have widely been known to reduce the formation of free-radicals. However to achieve good efficiency high levels of these materials have to be used and this can result in dark, aesthetically unpleasing products.

Acne vulgaris (acne) is a chronic inflammatory condition of the pilosebaceous units of the skin, which is particularly prevalent in adolescents. The condition generally causes the formation, on the skin, of comedones, red papules, pustules and sometimes cysts. This is unsightly and furthermore, if untreated, acne can lead to scarring of the skin.

The main reasons for the lesion development are hyperkeratosis of the sebaceous follicle and the increase of sebum secretion, and the prepubertal increase in sebum secretion which is associated with great changes in sebum composition. It seems possible that changes in the composition of sebum may be more important than increases in the amount of sebum in contributing to comedogenesis. These changes in the sebum composition are mainly due to lipid peroxidation.

Lipid peroxidation is mainly due to two factors:

### 1. Sebum oxidation

Sebum is composed of a great number of lipids such as squalene, cholesterol, cholesterol esters, wax esters and triglycerides plus free fatty acids and mono- and diglycerides (residues of the hydrolysis of the triglycerides by *Propionibacterium acnes).*

Squalene is one of these lipids and is specific to human sebum. Its oxidation generates peroxides which exert comedogenic effects. In open and closed comedones a high content of polar lipids (such as squalene peroxides) can be found. Squalene oxidation is thought to be the link between comedogenesis and bacterial colonisation.

It has been demonstrated that the peroxidation of sebum is probably linked with inflammation of the comedones.

Linoleic acid is also a component of the sebum. It has been shown that a relative lack of linoleic acid (due to its peroxidation) has an important role in the comedogenesis.

### 2. Propionibacterium acnes (P.acnes) increases directly and indirectly lipid peroxidation

*P. acnes* liberates an extracellular lipase which hydrolyses triglycerides in free fatty acids (oxidised lipids). From this degradation, *P. acnes* gains nutritional advantage.

Indirectly, *P. acnes* proliferates in the mixture of sebum and follicular cells resulting in the production of inflammatory compounds with neutrophil chemotaxis. Neutrophils produce reactive oxygen species for expelling the microorganisms. Excessive generation of reactive oxygen species may enhance the harmful destruction of surrounding healthy tissues.

Products that are active against *P. acnes* are known to be useful in the treatment of acne. Antioxidants are used in skincare compositions to reduce the formation of free-radicals, but the use of an antioxidant complex in acne skincare products will also help to prevent sebum oxidation and will therefore contribute to fighting acne. Compositions containing both an antimicrobial active and one or more antioxidant(s) are clearly of benefit for curing or mitigating the effects of acne, and reducing photoageing. A safe and efficacious agent that affords both antimicrobial and antioxidant properties would provide an additional advantage.

The anti-acne actives that are currently used in topical products are often harsh and drying, and many have well-known adverse effects linked to their method of action. For example, benzoyl peroxide works by killing the bacteria that cause inflammation, but side-effects include hypersensitivity, irritation and contact dermatitis. Salicylic and glycolic acids are frequently included in anti-acne compositions to unblock pores and stimulate new cell growth but, as for other alpha-hydroxy acids and beta-hydroxy acids, skin reddening and irritation may occur. Witch hazel is an astringent which dries out spots, but also dries the surface of the skin. Also, antibiotics are used topically to treat acne. Antibiotics reduce the number of bacteria on the skin's surface and in the follicles, and have an anti-inflammatory action. However, skin irritation is a common side-effect, some users develop contact dermatitis due to irritancy or allergy, and bacterial resistance frequently arises with intermittent use of topical antibiotics.

Thus, there exists a need for an effective anti-acne active which is gentler to the skin than current actives and, more preferably, an anti-acne active that also has anti-inflammatory properties and/or soothing properties a) to calm inflamed problem skin, and b) to counteract the adverse effects of any additional anti-acne actives that may be used in a formulation.

Essential oils are used extensively in a wide range of products, eg in aromatherapy, perfumery, foods, cleaning materials and as solvents. The use of essential oils in cosmetic and toiletry compositions is also well known. However, there are toxicological concerns associated with the use of essential oils on the skin. Essential oils are often skin irritants at relatively low concentrations; Tea tree oil and spearmint oil are common examples.

Dandruff is the most common condition affecting the scalp. Dandruff occurs when the process of skin renewal speeds up so that a greater number of dead cells are shed from the surface of the skin. The scalp becomes scaly and the skin cells collect in clumps so that they become noticeable when brushing the hair and may gather on the shoulders. Current anti-dandruff actives include soluble actives, eg piroctone olamine (octopirox) and the substituted imidazolyls ketoconazole and climbazole; particulate crystalline agents, eg sulphur, selenium disulphide and pyridinethione salts such as zinc pyrithione; and coal tar, which works by reducing the level of the yeast *Pityrosporum ovale* (*P. ovale*). Dandruff responds well to these treatments but can commonly recur if treatment is stopped. Therefore many dandruff sufferers have to use anti-dandruff products frequently, over long periods of time. Current anti-dandruff products are often drying and many, particularly those containing coal tar, have a dark colour and an unpleasant, pungent odour. The particulate anti-dandruff actives have additional disadvantages. They have a tendency to settle and separate and hence require the use of a suspending agent to maintain stability. Furthermore, there may be patchy or insufficient deposition of the active onto the scalp.

Many consumers would prefer to use products containing a natural anti-dandruff ingredient, that are pleasant to use, aesthetically pleasing once applied and less drying than the synthetic alternatives. A natural anti-dandruff active which is not drying and also soothes the scalp, and has antioxidant properties to further protect the hair from damage, would be of considerable benefit.

We have now surprisingly found that *Myrica gale* oil is of utility when applied topically to the skin or other exposed surfaces of the body, in particular that it exhibits antioxidant, antimicrobial properties and/or soothing/anti-inflammatory properties when so used. Furthermore, *Myrica gale* oil is a safe and non-irritant essential oil at surprisingly high concentrations.

According to the first aspect of the invention, there is provided the use of *Myrica gale* oil in the manufacture of a composition for the treatment of the skin or other exposed surfaces of the body.

By "for the treatment of the skin" is meant in this context the provision of a direct therapeutic and/or cosmetically beneficial effect upon the skin. Such an effect may be the prevention, alleviation or cure of a skin disorder, eg acne or dandruff. Alternatively, the effect may be a cosmetic effect, improving the appearance of the skin, eg by diminishing, diminishing the appearance of, and/or inhibiting the formation of, fine lines or wrinkles on the skin. This is in contrast to known prior uses of *Myrica gale* when applied to the skin, in which the *Myrica gale* serves as an insect repellent but was not considered to exert any beneficial effect on the skin *per se*.

Compositions prepared in accordance with the invention are advantageous primarily as anti-acne, anti-ageing, anti-dandruff and skin-soothing products. *Myrica gale* may be used with other active agents which have complementary benefits, or which work synergistically with *Myrica gale* oil to produce a particularly efficacious product.

By "soothing" in this context is meant the amelioration of skin discomfort. Skin discomfort can take several forms. Examples include dry, taut skin, uncomfortable itchiness, prickling sensations, and stinging sensations. Any of these may occur with or without reddening. When one or more of these is present, especially with reddening, the skin may be described as inflamed.

Tests to assess the soothing or anti-inflammatory properties of any agent may involve quantifiable insults to the skin, such as a defined ultraviolet light dose or defined exposure to an irritant such as lactic acid or sodium lauryl sulfate. The measures of soothing or anti-inflammatory capacity use either sensory self-assessment or objective, biophysical endpoints such as cytokine release in cell culture, or redness and blood flow *in vivo*.

The concentration of *Myrica gale* oil in compositions prepared in accordance with the invention will generally be in excess of 0.001 % by weight, more commonly in excess of 0.1 % by weight, and preferably in excess of 0.2% by weight. The concentration of *Myrica gale* oil is preferably less than 50% by weight, more preferably less than 30% by weight, more preferably less than 10% by weight and most preferably less than 5% by weight. The concentration of *Myrica gale* oil may therefore fall in the range 0.001 % to 50% by weight, more preferably 0.1 % to 10% by weight and most preferably 0.2% to 5% by weight.

The *Myrica gale* oil is preferably obtained by steam distillation and/or water distillation, supercritical carbon dioxide extraction, supercritical water or subcritical (superheated) water extraction, most preferably by subcritical water extraction.

In general, the *Myrica gale* oil will consist of a large number of different compounds, commonly in excess of 100 compounds. A large proportion of the components of the oil are normally terpenes, particularly monoterpenes and sesquiterpenes. The principal components of the oil, eg many of the compounds that make up more than 3% w/w of the oil, normally include a number of monoterpenes. Examples of monoterpenes that are commonly present include α-pinene, limonene, 1,8-cineole, α-phellandrene and p-cimene.

It is preferable that specific measures are taken to ensure that the oil does not undergo significant oxidative degradation during production or storage. These measures may include the use of brown glass or aluminium containers; that the containers are filled to capacity, nitrogen flushed and sealed immediately after distillation; and the addition of an antioxidant to each batch. Examples of suitable antioxidants include butylated hydroxytoluene (BHT), Tinoguard TT or tocopherol-containing antioxidant products, such as Controx. To avoid oxidative degradation, the oil is preferably prepared in a form that is free, or substantially free, from water.

Compositions prepared in accordance with the invention may be formulated in any one of numerous different forms. Suitable types of composition, and methods by which they may be prepared, will generally be evident to those skilled in the art.

One preferred group of compositions are formulated as emulsions. The emulsions may be o/w, w/o, o/w/o or w/o/w emulsions. Such emulsion-type compositions, which may be described *inter alia* as creams or lotions, are believed to be novel, and represent a further aspect of the invention, according to which there is provided a composition suitable for application to the skin or other exposed surface of the body, which composition comprises *Myrica gale* oil and is in the form of an emulsion. Preferred emulsion compositions are o/w emulsions.

Many compositions prepared in accordance with the invention comprise a one or more surfactants, in addition to the *Myrica gale* oil. Such compositions too are believed to be novel, and represent a further aspect of the invention, according to which there is provided a composition suitable for application to the skin or other exposed surface of the body, which composition comprises *Myrica gale* oil and one or more surfactants. Compositions comprising relatively high levels of surfactant, eg more than 10%, more than 20% or more than 30% w/w of surfactant, may be of particular utility in haircare products, eg as shampoos.

Some compositions prepared in accordance with the invention contain a lower alcohol (eg ethanol or isopropyl alcohol), in addition to the *Myrica gale* oil. Such compositions are believed to be novel and represent a further aspect of the invention, according to which there is provided a composition suitable for application to the skin or other exposed surface of the body, which composition comprises *Myrica gale* oil and one or more lower alcohols. Ethanol is the most commonly used lower alcohol. Such compositions may take the form of gels or lotions, eg lotions of the type known as toners.

Still further compositions prepared in accordance with the invention may be solid or semi-solid in form. Such compositions may be characterised by the presence in the composition of particulate inorganic material. Again such compositions are believed to be novel, and according to a further aspect of the invention there is provided a composition suitable for application to the skin or other exposed surface of the body, which composition comprises *Myrica gale* oil in admixture with one or more inorganic materials in particulate form. Examples of particlulate materials that may be used include talc, chalk and mica.

The composition may additionally comprise one or more active agents, which increase the efficacy of the composition either by working as a synergistic combination with the *Myrica gale* oil, or by providing a complementary benefit. Whatever the form of such compositions, they are believed to be novel. Thus, according to a yet further aspect of the invention, there is provided a composition suitable for application to the skin or other exposed surface of the body, which composition comprises *Myrica gale* oil and one or more other active ingredients.

Examples of other active ingredients that may be included in the composition, in addition to *Myrica gale,* include herbal extracts, desquamatory actives, anti-acne actives, vitamin B3 compounds, retinoids, di-, tri-, tetra- and penta- peptides and derivatives thereof, hydroxy acids, antioxidants, chelators, anti-inflammatory agents, topical anaesthetics, tanning actives, skin lightening agents, anti-cellulite agents, flavenoids, antimicrobial actives, skin healing agents, antifungal actives, farnesol, phytantriol, allantoin, glucosamine and mixtures thereof.

Preferred examples of one or more additional antioxidants suitable for inclusion in the composition include:
a) Vitamin C (ascorbic acid) its salts, esters, glucosides and glucosamines, particularly sodium ascorbyl phosphate, magnesium ascorbyl phosphate and ascorbyl palmitate, and other sources eg Acerola cherry powder.
b) Vitamin E (tocopherol) and its esters, particularly tocopheryl acetate.
c) Other vitamins and minerals eg Coenzyme Q10, selenium, magnesium, copper and zinc.
d) Carotenoids eg beta-carotene, lutein and lycopene.
e) Polyphenolics eg from *Camellia sinensis* (Green Tea), *Pyrus malus* (apple), rosamarinic acid and pycnogenol.
f) Herbal extracts eg G*ingko biloba, Morus alba* (mulberry), *Origanum vulgare* (oregano), *Panax ginseng* (ginseng), *Rosmarinus officinalis, Salvia officinalis* (sage), *Ziziphus spina-christi* and *Vitis vinifera* (grape seed).
g) Synthetic antioxidants such as butylated hydroxytoluene (BHT) and butylated hydroxyanisole (BHA).

More preferred antioxidants include sodium and magnesium ascorbyl phosphate, *Panax ginseng, Morus alba, Origanum vulgare* and *Rosmarinus officinalis* extracts.

In combination, the total amount of additional antioxidant agents (ie not including *Myrica gale* oil) may range from 0.001 % to 10% by weight, preferably 1 % to 7% by weight and more preferably 2% to 5% by weight of the composition.

Preferred examples of herbal extracts that may be used in combination with *Myrica gale* to provide a complementary benefit are *Aesculus hippicastanum* (horsechestnut), *Equisetum arvense* (horse tail), *Arctium lappa* (burdock), *Ruscus aculeatus* (box holly), *Vitis vinifera* (grape), *Salix alba* (willowbark) and *Betula pendula* (silver birch) extract.

Preferred additional anti-acne actives are salicylic acid, witch hazel, benzoyl peroxide, sulfur, sodium sulfacetamide, triclosan, glycolic acid, tea tree oil, rosemary, burdock and willow extract.

The composition may additionally comprise one or more additional anti-dandruff actives, eg zinc pyrithione, piroctone olamine (octopirox), sulphur, selenium disulphide, ketoconazole, climbazole or coal tar.

The composition may additionally comprise ore or more antimicrobial or antibacterial compounds, for example selected from the following:
triclosan, neomycin, clindamycin, polymyxin, bacitracin, benzoyl peroxide, tetracylines such as doxycycline or minocycline, sulfa drugs such as sulfacetamide, penicillins, cephalosporins such as cephalexin, and quinolones such as lomefloxacin, olfoxacin or trovafloxacin.

The composition may additionally comprise one or more anti-inflammatory compounds, for example selected from the following: aloe vera gel, aloe vera, licorice extract, pilewort, Canadian willow root, zinc, and allantoin.

The composition will generally comprise other ingredients or excipients which constitute or form part of the dermatologically acceptable carrier and will be well known to those skilled in the art. These include, for example:
a) Humectants, eg glycerin, propylene glycol, butylene glycol, hexylene glycol, dipropylene glycol, polyethylene glycol, sorbitol, urea, xylitol, lactitol, lactic acid and salts, fructose, glucose, mannose, xylose, honey, pyrrolidone, and carboxylic acid and salts.
b) Emollients, eg PPG-15 stearyl ether, ethylhexyl stearate, cetyl dimethicone, octyldodecanol, PPG-20 methyl glucose ether, isopropyl myristate isopropyl palmitate, isopropyl laurate isodecyl laurate, isodecyl neopentanoate, isohexadecane, pentaerythrityl tetraisostearate, caprylic/capric triglyceride, canola oil, sunflower oil (Helianthus annuus), olive oil (*Olea europea*), cottonseed oil (*Gossypium herbaceum*), jojoba oil (*Simmondsia chinensis*), shea butter (*Butyrospermum parkii*), cocoa butter (*Theobroma cacao*), cupuacu butter (*Theobroma grandiflorum*), avocado oil (*Persea gratissima*), liquid paraffin, dimethicone, phenyl trimethicone, cyclopentasiloxane, dimethiconol and petrolatum.
c) Surfactants - anionic surfactants, eg sodium lauryl sulphate, sodium laureth sulphate, ammonium laureth sulphate, disodium laureth sulfosuccinate and sodium C12-15 pareth sulphate; amphoteric/zwitterionic surfactants, eg cocamidopropyl betaine, sodium cocoamphoacetate and cocamidopropyl hydroxysultaine; nonionic surfactants, eg laureth-3, oleth-5, cocamide DEA, cocamide MEA, PEG-5 cocamide, polysorbate 20, PEG-40 hydrogenated castor oil; and cationic surfactants, eg cetrimonium chloride, behentrimonium chloride and benzalkonium chloride.
d) Emulsifiers, eg steareth-2, steareth-21, steareth-10, ceteareth-5, ceteareth-12, ceteareth-20, oleth-10, glyceryl stearate, polyglyceryl-3 oleate, polyglyceryl-3 methylglucose distearate, sodium cetearyl sulphate, sodium stearate, PEG-12 Oleate, PEG-2 stearate, PEG-12 stearate, PEG-80 sorbitan, sorbitan oleate, sorbitan palmitate and cetyl PEG/PPG-10/1 dimethicone.
e) Chelating agents or sequestering agents (sequestrants) - ingredients that have the ability to complex with and inactivate metallic ions in order to prevent their adverse effects on the stability or appearance of the composition. Examples of chelating agents are ethylenediamine tetraacetic acid and its salts, notably the dipotassium and especially the disodium or tetrasodium salt.
f) Sunscreening agents - inorganic sunscreening agents, eg microfine titanium dioxide, microfine zinc oxide, iron oxides, talcs and boron nitride;
   and/or organic sunscreening agents, eg p-aminobenzoic acids, esters and derivatives thereof, for example, 2-ethylhexyl p-dimethylaminobenzoate and the octyl ester of p-aminobenzoic acid;
   methoxycinnamate esters such as 2-ethylhexyl p-methoxycinnamate, 2-ethoxyethyl p-methoxycinnamate or α,β-di-(p-methoxycinnamoyl)-α'-(2-ethylhexanoyl)-glycerin; benzophenones such as oxybenzone;
   2-phenylbenzimidazole-5-sulfonic acid and disodium phenyl dibenzimidazole tetrasulfonate and terphthalylidene dicamphor sulfonic acid; alkyl-β,β-diphenylacrylates for example alkyl α-cyano-β,β-diphenylacrylates such as octocrylene; triazines such as 2,4,6-trianilino-(p-carbo-2-ethylhexyl-1'-oxy)-1,3,5 triazine and bis-octyloxyphenol methoxyphenyl triazine; camphor derivatives such as methylbenzylidene camphor; organic pigment sunscreening agents such as methylene bis-benzotriazole tetramethyl butylphenol; silicone derivatives such as drometrizole trisiloxane, benzylidene malonate polysiloxane and dimethicodiethyl benzal malonate, salicylates such as octyl salicylate.
g) Preservatives - ingredients which prevent or retard microbial growth and thus protect the composition from spoilage. Examples of preservatives include DMDM hydantoin, propylparaben, methylparaben, phenoxyethanol, sodium benzoate, bronopol, sodium dehydroacetate, polyhexamethylenebiguanide hydrochloride, isothiazolone and diazolidinylurea.
h) Perfumes and colourings.

According to another aspect of the invention, there is provided a method for treating the skin or other exposed surface of the body, which method comprises the application to the skin or other exposed surface of a skincare composition comprising *Myrica gale* oil.

The treatment of the skin may be a prophylactic or remedial treatment of signs of ageing, eg the treatment of lines or wrinkles in the skin. In other embodiments, the treatment may be a prophylactic or remedial treatment of acne. Thus, in specific aspects of the invention, there are provided:
a) a method for the prophylactic or remedial treatment of acne, which method comprises the application of a skincare composition comprising *Myrica gale* oil to the skin of a patient suffering from, or susceptible to, acne; and
b) the use of *Myrica gale* oil in the manufacture of a composition for the prophylactic or remedial treatment of acne.

As described above, the compositions according to the invention may also be effective in the prophylactic or remedial treatment of dandruff. Thus, in further specific aspects of the invention, there are provided:
a) a method for the prophylactic or remedial treatment of dandruff, which method comprises the application of a composition comprising *Myrica gale* oil to the scalp of a patient suffering from, or susceptible to, dandruff; and
b) the use of *Myrica gale* oil in the manufacture of a composition for the prophylactic or remedial treatment of dandruff.

The invention will now be described in greater detail, by way of illustration only, with references to the following Examples.

### Example 1 - Skin Treatment Gel

| Ingredient | % |
|---|---|
| Alcohol (denatured) | 10.00 |
| Allantoin | 0.10 |
| Glycerin | 1.00 |
| Butylene glycol | 4.00 |
| Xanthan gum | 1.00 |
| Phenoxyethanol | 0.20 |
| Hydrated silica | 0.50 |
| Dichlorobenzyl alcohol | 0.10 |
| Colour | qs |
| Benzophenone - 4 | 0.10 |
| Purified water | to 100 |
| Panthenol | 0.50 |
| *Myrica gale* oil | 2 |

### Method

Xanthan gum, dispersed in 2% of butylene glycol was added to some of the purified water, and mixed together for 30 minutes. Allantoin, sequestrene and panthenol were added and the mixture was stirred for 5 minutes. The mixture was cooled to 35ºC, and then premixed phenoxyethanol and glycerin was added to the mixture, followed by premixed alcohol (denatured) and purified water, followed by premixed dichlorobenzyl alcohol and butylene glycol. The mixture was then stirred. Benzophenone-4 and water were then added with stirring, followed by hydrated silica. The mixture was stirred, cooled to below 35ºC and then the colour and *Myrica gale* oil were added. Cold water was added to the bulk and the mixture was stirred for a further 30 minutes.

### Example 2 - Non-oily Moisturiser

| Ingredients | % |
|---|---|
| Colour | |
| Perfume | 0.10 |
| Triclosan | 0.10 |
| Allantoin | 0.10 |
| Phenoxyethanol | 0.20 |
| Hydroxyethylcellulose | 2.00 |
| Polysorbate 20 | 1.00 |
| Butylene glycol | 3.50 |
| Glycerin | 4.50 |
| *Myrica gale* oil | 3.5 |
| Purified water | to 100 |

### Method

### Stage 1

Hydroxyethylcellulose was added to purified water and then homogenised for at least 30 minutes. The homogeniser was switched off and with stirring allantoin and phenoxyethanol, which had been previously dissolved in glycerin and butylene glycol, were added.

### Stage 2

Butylene glycol and glycerine were warmed together to 45ºC. Then with stirring, triclosan was added, stirred and completely dissolved and cooled to 35ºC.

### Stage 3

Using a homogeniser, stage 2 was added to stage 1 and homogenised for 10 minutes. The perfume, previously dispersed in polysorbate 20 was then added and stirred in well. The colour and *Myrica gale* were added and the emulsion was then homogenised for a further 5 to 10 minutes until the product was smooth. Purified water, sufficient to make the formulation up to bulk, was added.

### Example 3 - Cleansing Lotion

| Ingredients | % |
|---|---|
| Purified Water | to 100 |
| Colours | qs |
| *Melaleuca alternifolia* | 0.50 |
| Polypropylene glycol-5-ceteth-20 | 3.25 |
| Polysorbate 80 | 0.20 |
| Citric acid | 0.12 |
| Disodium phosphate | 0.38 |
| Triclosan | 0.30 |
| Butylene glycol | 0.20 |
| Alcohol (denatured) | 48.00 |
| *Myrica gale* oil | 7 |

### Method

### Stage 1

Alcohol (denatured) and triclosan were mixed together until homogeneous. Water was added and mixed well. Butylene glycol was then added and the mixture stirred.

### Stage 2

To a suitable stainless steel container disodium phosphate and water were added and warmed to 55-60ºC with stirring. Some water was added, with stirring, and the mixture was allowed to cool.

### Stage 3

When the temperature of stage 2 had reached 20-25ºC it was added to stage 1 with stirring. Citric acid was added and mixed well. To a suitable stainless steel container polypropylene glycol-5-ceteth-20 and *Melaleuca alternifolia* were added. This was then mixed thoroughly and added to the main vessel. In a suitable container polypropylene glycol-5-ceteth-20 and polysorbate 80 were premixed and this was then added to the main vessel. Sufficient colour was added, followed by the *Myrica gale* oil and then the water to make up to bulk.

### Example 4 - Cleansing Wash

| Ingredients | % |
|---|---|
| Cocamidopropyl betaine 30% | 5.00 |
| Benzophenone-4 | 0.10 |
| Sodium citrate | 0.60 |
| Disodium undecylenamido MEA- | |
| sulfosuccinate solution | 1.00 |
| Triclosan | 0.20 |
| Salt | 1.00 |
| Laureth-3 | 2.00 |
| Sodium laureth sulphate | 47.20 |
| Colour | qs |
| *Melaleuca alternifolia* | 0.500 |
| Phenoxyethanol | 0.15 |
| Purified water | to 100 |
| Citric acid | 0.10 |
| *Myrica gale* oil | 8 |

### Method

### Stage 1

In a vessel sodium laureth sulphate, *Melaleuca alternifolia* and *Myrica gale* oil were mixed until uniform.

### Stage 2

In the base pan the triclosan was dispersed in cocamidopropyl betaine 30% and stirred for 5 minutes. The purified water was added and stirred well. Citric acid was added and stirred until dissolved followed by sodium citrate, which was stirred until dissolved and then stirred for a further 10 minutes. The mixture was cooled to 30-35ºC. Then premixed phenoxyethanol in water, followed by benzophenone-4 in water were added. The colour was added followed by saline solution and water to make up to bulk.

### Example 5 - Moisture Fluid

| Ingredients | % |
|---|---|
| Glyceryl stearate | 1.50 |
| Steareth-2 | 2.00 |
| Steareth-21 | 1.00 |
| Cetyl alcohol | 1.00 |
| Glycerin | 1.00 |
| Butylene glycol | 2.00 |
| Purified water | 90 |
| *Myrica gale* oil | 1.5 |

### Stage 1

Glyceryl stearate, steareth-2, steareth-21 and cetyl alcohol were melted together at 70-75ºC.

### Stage 2

Glycerin was dissolved with stirring in water at 70-75ºC.

### Stage 3

Stage 1 was then added to stage 2 with stirring and then homogenised for 15 minutes. Water was added to the stirred mixture, which was subsequently cooled to 35ºC. Butylene glycol and *Myrica gale* oil were added and the mixture stirred until homogeneous and then made up to bulk with water.

### Example 6 - Dandruff Shampoo

| Ingredients | % |
|---|---|
| Magnesium aluminium silicate | 0.30 |
| Polyacrylic acid solution | 1.50 |
| Purified water | 62.10 |
| Preservative | 0.08 |
| Salt | 1.00 |
| Citric acid | 0.02 |
| Cocamidopropyl betaine 50% | 5.00 |
| Sodium laureth sulphate | 30.0 |
| *Myrica gale* oil | 0.6 |

### Method

Citric acid, salt and preservative were dissolved in water. Polyacrylic acid solution and magnesium aluminium silicate were added and the mixture homogenised. The mixture was then stirred for 20 minutes. Sodium laureth sulphate, cocamidopropyl betaine and *Myrica gale* oil were added, followed by sufficient salt to obtain the correct viscosity.

### Example 7 - Mens Facial Wash

| Ingredients | % |
|---|---|
| Hydroxyethylcellulose | 1.25 |
| Sodium laureth sulfate | 6.57 |
| Disodium undecylenamido MEA-sulfosuccinate | 1.00 |
| Butylene glycol | 2.00 |
| Preservative | 0.80 |
| Benzoic acid | 0.10 |
| Polysorbate-20 | 2.00 |
| Perfume | 0.40 |
| Herbal extract | 0.60 |
| Colour | qs |
| *Myrica gale* oil | 2.5 |
| Purified Water | to 100 |

### Method

### Stage 1

Butylene glycol and preservative were put in a stainless steel vessel and then mixed until uniform.

### Stage 2

Perfume, polysorbate-20 and *Myrica gale* oil were put in another container and mixed until uniform.

### Stage 3

Hydroxyethylcellulose was mixed with some of the water in a steel container for 20-30 minutes until fully dispersed. Stage 1 was added, mixed and then the herbal extract, benzoic acid and colour were added and then mixed until fully dispersed.

### Stage 4

Sodium laureth sulfate, and disodium undecylenamdo MEA-sulfosuccinate were added to stage 2 and mixed. Stage 2 was then added to stage 3 and mixed thoroughly. Cold water was added to make up to bulk. The bulk was stirred carefully to prevent foaming.

### Example 8 - Translucent Complexion Base

| Ingredients | % |
|---|---|
| Triethanolamine pure solution 80% | 1.50 |
| Methylparaben | 0.20 |
| Polyethylene glycol-5-ceteth-20 | 0.60 |
| Allantoin | 0.10 |
| Hydrated silica | 12.00 |
| Stearic acid | 12.00 |
| Cetyl alcohol | 1.20 |
| Butylene glycol | 10.00 |
| Propylparaben | 0.10 |
| Dicaprylyl maleate | 4.00 |
| Alcohol denatured | 2.00 |
| Sodium C8-16 isoalkylsuccinyl lactoglobulin sulfonate | 1.00 |
| Alpha-glucan oligosaccharide | 0.20 |
| Herbal extract | 0.40 |
| Purified Water | 51.68 |
| Octyl palmitate | 3.00 |
| Butylated hydroxytoluene | 0.02 |
| *Myrica gale* oil | 0.5 |

### Method

### Stage 1

Butylene glycol, allantoin, polypropylene glycol-5-ceteth 20 and methylparaben were added, whilst stirring, to the water at 70-75ºC. Hydrated silica was then gradually added and mixed until uniform.

### Stage 2

Stearic acid, cetyl alcohol, octyl palmitate, dicaprylyl maleate were melted together at 70-75ºC. Using a silverson, butylated hydroxytoluene, propylparaben and triethanolamine pure solution 80% were added and the mixture stirred for 5 minutes.

### Stage 3

Both stages were warmed to 70-75ºC, and then stage 2 was added to stage 1 and stirred for 5-10 minutes. The mixture was cooled to 40ºC with stirring, and then dicaprylyl maleate was added. The mixture was cooled to 30ºC then alpha-glucan oligosaccharide in water, denatured alcohol, sodium C8-16 isoalkylsuccinyl lactoglobulin sulfonate, herbal extracts including *Myrica gale* oil, and water were added to the mixture. The mixture was then stirred until uniform.

### Example 9 - Translucent Complexion Base

| Ingredients | % |
|---|---|
| High surface area zinc oxide | 4.00 |
| Purified water | 46.68 |
| Triethanolamine pure solution 80% | 1.50 |
| Methylparaben | 0.20 |
| Polypropylene glycol-5-ceteth -20 | 0.60 |
| Allantoin | 0.10 |
| Hydrated silica | 12.00 |
| Stearic acid | 12.00 |
| Cetyl alcohol | 1.20 |
| Butylene glycol | 10.00 |
| Propylparaben | 0.10 |
| Dicaprylyl maleate | 4.00 |
| Alcohol denatured | 2.00 |
| Sodium C8-16 isoalkylsuccinyl lactoglobulin sulfonate | 1.00 |
| Alpha-glucan oligosaccharide | 0.20 |
| Herbal extract | 0.40 |
| Octyl palmitate | 3.00 |
| Butylated hydroxytoluene | 0.02 |
| *Myrica gale* oil | 1 |

### Method

### Stage 1

Butylene glycol, allantoin, polypropylene glycol-5-ceteth 20 and methylparaben were added, whilst stirring, to the water at 70-75ºC. Hydrated silica and high surface area zinc oxide was then gradually added and mixed until uniform.

### Stage 2

Stearic acid, cetyl alcohol, octyl palmitate, dicaprylyl maleate were melted together at 70-75ºC. Using a silverson, butylated hydroxytoluene, propylparaben and triethanolamine pure solution 80% were added and the mixture stirred for 5 minutes.

### Stage 3

Both stages were warmed to 70-75ºC, and then stage 2 was added to stage 1, and stirred for 5-10 minutes. The mixture was cooled to 40ºC with stirring, and then dicaprylyl maleate was added. The mixture was cooled to 30ºC then alpha-glucan oligosaccharide in water, denatured alcohol, sodium C8-16 isoalkylsuccinyl lactoglobulin sulfonate, herbal extracts, including *Myrica gale* oil, and water were added to the mixture. The mixture was then stirred until uniform.

### Example 10 - Pressed Powder

| Ingredients | % |
|---|---|
| Sanitised talc | 90.72 |
| Magnesium stearate | 5.00 |
| Methylparaben | 0.10 |
| Red colour | 0.23 |
| Yellow colour | 0.45 |
| Paraffinum liquidum | 1.80 |
| Petrolatum | 1.40 |
| *Myrica gale* oil | 0.3 |

### Method

Sanitised talc, magnesium stearate, methylparaben, *Myrica gale* oil and colours were mixed together for 10 minutes at high speed. Paraffinum liquidum and petrolatum were mixed together, heated to 75ºC then sprayed into the bulk mixture at low speed. The bulk was mixed for 5 minutes, and then passed twice through a hammer mill before being passed through a 30 mesh sieve.

### Example 11 - Cover Up Stick

| Ingredient | % |
|---|---|
| Butylated hydroxyacetone | 0.03 |
| Carnauba | 1.29 |
| Candelilla Cera | 1.01 |
| Hydrocarbon wax consisting of Cera | |
| Microcristallina, paraffin and | |
| polyethylene | 4.60 |
| Cera Microcristallina | 4.22 |
| Synthetic wax | 2.01 |
| Propylparaben | 0.10 |
| Chalk | 18.73 |
| Triclosan | 0.19 |
| Allantoin | 0.14 |
| Pigment | 21.56 |
| Octyldodecanol | to 100 |
| *Myrica gale* oil | 0.2 |

### Method

Pigments and chalk were added to the Diosna mixer and mixed for 30 minutes. The mix was then passed through the Mikro mill, then a vibrating sieve to give the colour preparation.

White wax, Carnauba and candelilla cera were added to a stainless steel steam jacketed pan fitted with a premier dispersator head, and melted together at 90-95ºC. To the melt was added the hydrocarbon wax. When melted, ocyldodecanol was added and the mixture stirred.

The mixture was cooled to 85-90ºC then propylparaben, butylated hydroxyacetone and triclosan were added to the stirred mixture, followed by allantoin and then by the colour preparation. The mixture was then stirred for a further ten minutes.

The mixture was then stirred through a 40 mesh sieve into a shallow tray and stirred slowly until set.

### Example 12 - Cover Up Stick (With High Surface Area Zinc Oxide)

| Ingredient | % |
|---|---|
| Chalk | to 100 |
| Carnauba | 1.29 |
| Candelilla Cera | 1.01 |
| Hydrocarbon was consisting of Cera | |
| Microcristallina, paraffin and | |
| polyethylene | 4.60 |
| Cera Microcristallina | 4.22 |
| Butylated hydroxyacetone | 0.03 |
| Propylparaben | 0.10 |
| Octyldodecanol | 46.02 |
| Triclosan | 0.19 |
| Allantoin | 0.14 |
| Pigment | 11.98 |
| *Myrica gale* oil | 0.25 |
| Synthetic wax | 2.01 |

### Method

Pigments, high surface area zinc oxide and chalk were added to the Diosna mixer and mixed for 30 minutes. The mix was then passed through the Mikro mill, then a vibrating sieve to give the colour preparation.

White wax, Carnauba and candelilla cera were added to a stainless steel steam jacketed pan fitted with a premier dispersator head, and melted together at 90-95ºC. To the melt was added the hydrocarbon wax. When melted, ocyldodecanol was added and the mixture stirred.

The mixture was cooled to 85-90ºC then propylparaben, butylated hydroxyacetone and triclosan were added to the stirred mixture, followed by allantoin and then by the colour preparation. The mixture was then stirred for a further ten minutes.

The mixture was then stirred through a 40 mesh sieve into a shallow tray and stirred slowly until set.

### Example 13 - Baby Lotion Wipes

| Ingredient | % |
|---|---|
| Sodium citrate | 0.10 |
| Purified water | 75.03 |
| Perfume | 0.10 |
| Polyaminopropyl biguanide | 0.75 |
| 2-Bromo-2-Nitropropane-1,3-Diol | 0.02 |
| Cetrimonium bromide | 0.50 |
| A wax blend consisting of cetearyl | |
| alcohol, cetyl palmitate, | |
| cocoglycerides and glyceryl stearate | 0.50 |
| Steareth-10 | 1.50 |
| *Myrica gale* oil | 0.8 |
| An emulsifier blend consisting of | |
| glyceryl stearate and polyethylene | |
| glycol-30 stearate | 1.50 |
| Mineral oil | 19.20 |

### Method

### Stage 1

Steareth 10 was added to a base pan containing mineral oil at 70ºC. The emulsifier blend and the wax blend were then added to the stirred mixture and melted together at 70ºC.

### Stage 2

Cetrimonium bromide was added to purified water at 70ºC and mixed in a homogeniser.

### Stage 3

Stage 1 was then added to stage 2, homogenised and stirred. Cold purified water was added and the mixture force cooled to 35ºC. The perfume was then added, followed by *Myrica gale* oil, polyaminopropyl biguanide and a solution of 2-bromo-2- nitropropane-1,3-diol in cold water. Water was then added to make up to bulk.

### Example 14 - Baby Lotion Wipes (High Surface Area Zinc Oxide)

| Ingredient | % |
|---|---|
| Sodium citrate | 0.10 |
| Purified water | 69.43 |
| Perfume | 0.10 |
| Polyaminopropyl biguanide | 0.75 |
| 2-Bromo-2-nitropropane-1,3-diol | 0.02 |
| Cetrimonium bromide | 0.50 |
| A wax blend consisting of cetearyl | |
| alcohol, cetyl palmitate, | |
| cocoglycerides and glyceryl stearate | 0.50 |
| Steareth-10 | 1.50 |
| An emulsifier blend consisting of | |
| glyceryl stearate and polyethylene | |
| glycol-30 stearate | 1.50 |
| Mineral oil | 20.00 |
| High surface area zinc oxide | 5.00 |
| *Myrica gale* oil | 0.6 |

### Method

### Stage 1

Steareth 10 and high surface area zinc oxide was added to a base pan containing mineral oil at 70ºC. The emulsifier blend and the wax blend were then added to the stirred mixture and melted together at 70ºC.

### Stage 2

Cetrimonium bromide was added to purified water at 70ºC and mixed in a homogeniser.

### Stage 3

Stage 1 was then added to stage 2, homogenised and stirred. Cold purified water was added and the mixture force cooled to 35ºC. The perfume was then added, followed by *Myrica gale* oil, polyaminopropyl biguanide and a solution of 2-bromo-2-nitropropane-1,3-diol in cold water. Water was then added to make up to bulk.

### Example 15 - Nappy Rash Cream

| Ingredient | % |
|---|---|
| Cetostearyl alcohol | 2.00 |
| Zinc oxide | 7.50 |
| *Arachis hypogaena* | 30.50 |
| Cera alba | 10.00 |
| *Ricinus communis* | 47.00 |
| *Myrica gale* oil | 3 |

### Method

*Arachis hypogaena*, cera alba and cetostearyl alcohol were mixed together in a base pan at 65-70ºC. The mixture was then pumped through an 80 mesh sieve into a mixing vessel. Zinc oxide was added to the mixture which was stirred for 5 minutes until homogeneous. *Ricinus communis* and *Myrica gale* oil were then added to the mixture and the mixture stirred for 5 minutes until homogeneous.

### Example 16 - Nappy Cream

| Ingredient | % |
|---|---|
| Purified water | 39.40 |
| p-chloro-m-cresol | 0.10 |
| Cetrimonium bromide | 0.50 |
| Cetearyl alcohol | 5.00 |
| Paraffinum liquidum | 40.00 |
| Dimethicone | 10.00 |
| Zinc oxide | 4.5 |
| *Myrica gale* oil | 0.5 |

### Method

Cetrimonium bromide and p-chloro-m-cresol were added to a base pan containing water at 60-65ºC. A vacuum was applied to a fryma and the contents of the base pan transferred to the fryma via a sieve.

Dimethicone, paraffinum liquidum and cetearyl alcohol were added to the base pan, and warmed to 60-65□C. The contents of the base pan were then transferred to the fryma via a sieve. The contents of the fryma were stirred for 10 minutes. Maintaining the vacuum on the fryma, the stirred contents were cooled to 50-60ºC when high surface area zinc oxide was added. The mixture was then stirred until a smooth white cream was produced. The cream was then cooled to 35ºC and discharged to storage.

### Example 17- Shower Gel

| Ingredients | % |
|---|---|
| Purified Water | to100 |
| Perfume | 0.10 |
| Colour | 0.00075 |
| Salt | 1.19 |
| Dichlorobenzyl alcohol | 0.50 |
| Butylated hydroxytoluene | 0.0048 |
| Triclosan | 0.2916 |
| PEG - 7 glyceryl cocoate | 2.916 |
| Citric Acid | 0.0216 |
| A preservative blend consisting of: phenoxyethanol, | |
| butylparaben, ethylparaben, methylparaben and | |
| propylparaben | 0.80 |
| Cocamidopropyl betaine | 5.83 |
| Sodium laureth sulfate | 45.89 |
| *Myrica gale* oil | 0.5 |

### Method

Salt and citric acid were then added to the water and the mixture stirred until both had dissolved. Sodium laureth sulfate, cocamidopropyl betaine, phenoxyethanol, butylparaben, ethylparaben, methylparaben and propylparaben were then added and the mixture stirred.

PEG 7 glyceryl cocoate, butylated hydroxytoluene, triclosan and dichlorobenzyl alcohol were mixed together and warmed to 45ºC. *Myrica gale* oil and perfume were then added and the mixture stirred until homogenous.

The two mixtures were then combined and stirred until homogenous. Colour solution was added to the stirred mixture followed by water to make up to bulk. The mixture was then stirred until uniform.

### Example 18 - Acne Lotion

| Ingredients | % |
|---|---|
| Purified water | 81.115 |
| Benzoyl peroxide | 6.667 |
| Hydroxyethylcellulose | 1.00 |
| Citric acid | 1.53 |
| Sodium hydroxide | 0.6915 |
| Absorbent zinc oxide | 5.00 |
| *Myrica gale* oil | 1 |

### Method

Sodium hydroxide was added to a stirred aqueous solution of citric acid. Hydroxyethylcellulose was then added to the mixture which was then stirred for 30 minutes. Benzoyl peroxide was then added followed by some water. The mixture was stirred for 2 minutes then homogenised for 20 minutes under vacuum. High surface area zinc oxide was then added to the mixture and stirred thoroughly. Aqueous sodium hydroxide and *Myrica gale* oil were then added to the stirred mixture, which was then stirred for a further hour.

### Example 19 - Sensitive Cleansing Pads

| Ingredients | % |
|---|---|
| Purified Water | 66.52708 |
| A preservative blend consisting of: phenoxyethanol | |
| butylparaben, ethylparaben, methylparaben and | |
| propylparaben | 0.80 |
| Perfume | 0.05112 |
| Polysorbate-20 | 1.0224 |
| Sodium citrate | 0.2045 |
| Citric acid | 0.062 |
| Cetrimoniumbromide | 0.5112 |
| Alcohol (denatured) | 17.3806 |
| PPG-5-ceteth-20 | 0.3067 |
| Butylene glycol | 4.6008 |
| Glycerin | 1.0224 |
| Chlorhexidine digluconate | 0.5112 |
| Absorbent zinc oxide | 5.00 |
| *Myrica gale* oil | 2 |

### Method

Chlorhexidine gluconate, butylene glycol, PPG-5-ceteth-20, glycerin, cetriumoniumbromide, citric acid, sodium citrate were dissolved in purified water. Denatured alcohol, high surface area zinc oxide and the preservative blend were then added and the mixture stirred. A mixture of polysorbate-20, *Myrica gale* oil and perfume was then added and the mixture stirred. Purified water was added to make up to bulk. The mixture was stirred for 30 minutes then pumped through an 80 mesh sieve to a suitable storage vessel.

### Example 20 - Overnight Gel

| Ingredients | % |
|---|---|
| Purified water | 50.6 |
| Perfume | 0.05 |
| Triclosan | 0.10 |
| Dichlorobenzyl alcohol | 0.50 |
| Alcohol (denatured) | 39.50 |
| Hydroxyethylcellulose | 1.25 |
| Glycerin | 3.00 |
| *Myrica gale* oil | 5 |

### Method

Hydroxyethylcellulose and glycerin were dispersed in water then transferred to a fryma via a sieve covered with muslim.

Denatured alcohol, dichlorobenzyl alcohol and triclosan were mixed together until homogenous. Perfume and then *Myrica gale* oil were then dispersed in the mixture. The mixture was then transferred to the fryma under vacuum via a sieve. The mixture was stirred for 30 minutes until homogeneous. Water was then added to make up to bulk.

### Example 21 - Emergency Gel

| Ingredients | % |
|---|---|
| Purified water | 35.00 |
| Hydroxypropyl methylcellulose | 2.50 |
| Sodium citrate | 0.30 |
| Alcohol (denatured) | 20.00 |
| Butylene glycol | 15.00 |
| Propylene glycol | 18.00 |
| Triclosan | 0.20 |
| Salicylic acid | 2.00 |
| Absorbent zinc oxide | 4 |
| *Myrica gale* oil | 3 |

### Method

Propylene glycol, butylene glycol and ethanol were mixed together. Salicylic acid and triclosan were then dissolved in the mixture. High surface area zinc oxide and *Myrica gale* oil were then added to the stirred mixture. Hydroxypropyl methylcellulose was then dispersed in the mixture and the mixture was stirred for 30 minutes. Aqueous sodium citrate was then added to the mixture under vacuum and the mixture stirred for a further 30 minutes.

### Example 22 - Hair Conditioner

| Ingredients | % |
|---|---|
| Aqua | to 100 |
| Cetyl alcohol | 3 |
| Cetrimonium chloride | 0.759 |
| Hydroxyethylcellulose | 0.6 |
| Propylene glycol | 0.493 |
| Panthenol | 0.375 |
| Parfum | 0.3 |
| Benzophenone-4 | 0.2 |
| Sodium chloride | 0.15 |
| Wheat amino acids | 0.14 |
| Amodimethicone | 0.105 |
| Alcohol denat. | 0.095 |
| Dimethicone propyl PG-betaine | 0.09 |
| Citric acid | 0.026 |
| Tetrasodium EDTA | 0.02 |
| Trideceth-10 | 0.0045 |
| *Origanum vulgare* | 0.009 |
| *Myrica gale* oil | 5 |

### Method

The EDTA and hydroxyethylcellulose were added to the water and mixed using a homogeniser to hydrate the polymer. Citric acid, benzophenone and cetrimonium chloride were added. This was then heated to 70ºC.

Cetyl alcohol was heated to 70ºC in a separate vessel and was then added to the aqueous mixture using a homogeniser. The mixture was then cooled to below 40ºC using a propeller stirrer. The remaining materials including the *Myrica gale* oil were then added and the product was made to weight with purified water.

### Example 23 - Leave In Conditioner

| Ingredients | % |
|---|---|
| Aqua | to 100 |
| PEG-40 hydrogenated castor oil | 2 |
| Dipropylene glycol | 1 |
| Phenoxyethanol | 0.85 |
| Parfum | 0.4 |
| Panthenol | 0.375 |
| Propylene glycol | 0.29 |
| Methylparaben | 0.2 |
| Benzophenone-4 | 0.2 |
| Alcohol denat. | 0.095 |
| Polyquaternium-10 | 0.091 |
| Sodium chloride | 0.3 |
| Wheat amino acids | 0.028 |
| Sodium hydroxide | 0.026 |
| *Myrica gale* oil | 3 |

### Method

The polyquaternium-1 0 was added to the water and hydrated using a propeller stirrer. The methylparaben was pre-dispersed in the dipropylene glycol, gently heated to melt and then added to the hydrated polyquaternium-1 0 mixture. The remaining materials including the *Myrica gale* oil were then added and the product was mixed and made to weight with purified water.

### Example 24 - Gentle Shampoo

| Ingredients | % |
|---|---|
| Aqua | to 100 |
| Sodium laureth sulfate | 8.25 |
| Cocamidopropyl betaine | 2.8 |
| Sodium chloride | 1.794 |
| Cocamide DEA | 1.63 |
| PEG-6 cocamide | 1 |
| Parfum | 0.5 |
| Panthenol | 0.375 |
| Propylene glycol | 0.29 |
| Benzophenone-4 | 0.2 |
| Glycerin | 0.2 |
| Phenoxyethanol | 0.162 |
| Wheat amino acids | 0.14 |
| Alcohol denat. | 0.095 |
| Citric acid | 0.05 |
| Methyldibromo glutaronitrile | 0.04 |
| Tetrasodium EDTA | 0.02 |
| *Origanum vulgare* | 0.009 |
| *Morus alba* | 0.0046 |
| *Myrica gale* oil | 0.5 |

### Method

EDTA, sodium chloride, citric acid and benzophenone-4 were added to the water.

This was followed by the addition of sodium laureth sulfate, methyldibromo glutaronitrile, wheat amino acids and the antioxidant complex.

PEG-6 cocamide and cocamide DEA were heated gently until liquified. The parfum was added and mixed. This was then added to the above mixture. The cocamidopropyl betaine and remaining materials were then added and mixed. The product was made to weight using purified water.

### Example 25 - Anti-Dandruff Shampoo

| Ingredients | % |
|---|---|
| Aqua | to 100 |
| Sodium laureth sulfate | 5.9 |
| Disodium laureth sulfosuccinate | 4 |
| Laureth-3 | 3 |
| Cocamidopropyl betaine | 2.45 |
| Sodium chloride | 1.926 |
| Dipropylene glycol | 1 |
| Parfum | 0.5 |
| Piroctone olamine | 0.5 |
| Panthenol | 0.375 |
| Propylene glycol | 0.29 |
| Disodium phosphate | 0.25 |
| Benzophenone-4 | 0.2 |
| Wheat amino acids | 0.14 |
| Alcohol denat. | 0.095 |
| Citric acid | 0.063 |
| Tetrasodium EDTA | 0.02 |
| Preservative | 0.2 |
| *Myrica gale* oil | 10 |

### Method

EDTA, citric acid and benzophenone-4 were mixed into the water. Sodium laureth sulfate, disodium laureth sulfosuccinate, dipropylene glycol, disodium phosphate, wheat amino acids and the antioxidant complex were added and the product was stirred until uniform. The piroctone olamine was dispersed in the parfum and *Myrica gale* oil and added to the laureth-3. This mixture was added to the bulk and stirred. The remaining materials were then added and the product was made to weight with purified water.

### Example 26 - Hair Gel

| Ingredients | % |
|---|---|
| Aqua | to 100 |
| Cyclomethicone | 6.6 |
| Dimethiconol | 0.9 |
| Phenoxyethanol | 0.8 |
| Propylene glycol | 0.79 |
| Panthenol | 0.75 |
| Carbomer | 0.7 |
| Aminomethyl propanol | 0.4 |
| Benzophenone-4 | 0.2 |
| Parfum | 0.2 |
| Alcohol denat. | 0.095 |
| Tetrasodium EDTA | 0.05 |
| Sodium chloride | 0.03 |
| Wheat amino acids | 0.028 |
| *Myrica Gale* oil | 5 |

### Method

EDTA and benzophenone-4 were added to the water using an homogeniser. The carbomer was added and hydrated with continued homogenising. The phenoxyethanol, *Myrica gale* oil, cyclomethicone, dimethiconol, propylene glycol and panthenol were t mixed until homogenous and then added to the water phase. The remaining materials were added and the bulk was homogenised until uniform and the product was made to weight using purified water.

### Example 27 - Semi-solid Structured Styling Paste for Hair

This type of product is also known as hair "putty"

| Ingredients | % |
|---|---|
| Aqua | to 100 |
| Cetearyl alcohol | 10.9 |
| Lanolin | 7 |
| PVP | 6 |
| Paraffin | 6 |
| PVP/VA copolymer | 5.7 |
| Carnauba | 3 |
| Petrolatum | 2 |
| Polyquaternium-11 | 2 |
| PEG-20 stearate | 1.9 |
| Paraffinum liquidum | 1 |
| Propylene glycol | 0.8 |
| Phenoxyethanol | 0.6 |
| Dimethicone | 0.5 |
| Panthenol | 0.375 |
| Cetrimonium chloride | 0.35 |
| Dimethicone propyl PG-betaine | 0.225 |
| Benzophenone-4 | 0.2 |
| Methylparaben | 0.12 |
| Alcohol denat. | 0.095 |
| *Origanum vulgare* | 0.009 |
| *Panax ginseng* | 0.006 |
| *Morus alba* | 0.0046 |
| *Myrica gale* oil | 2 |

### Method

The PVP/VA copolymer, PVP and benzophenone-4 were added to the water and stirred until homogenous. This was then heated to 70ºC. In a separate vessel, the waxes were mixed and heated to 70ºC until all materials had melted.

The hot waxes were then added to the aqueous mixture and mixed using a propeller stirrer until homogenous. The mixture was then cooled to below 60ºC and the remaining materials, including the *Myrica gale* oil were then added and the product was stirred until uniform. The product was made to weight using purified water.

### Example 28 - Moisturising Conditioner

| Ingredients | % |
|---|---|
| Aqua | to 100 |
| Cetyl alcohol | 4 |
| Dimethicone | 2 |
| Hydroxyethylcellulose | 0.8 |
| Cetrimonium chloride | 0.765 |
| Panthenol | 0.75 |
| Propylene glycol | 0.64 |
| Parfum | 0.3 |
| Benzophenone-4 | 0.2 |
| Amodimethicone | 0.175 |
| Dimethicone propyl PG-betaine | 0.15 |
| Sodium chloride | 0.15 |
| Wheat amino acids | 0.14 |
| Alcohol denat. | 0.095 |
| Citric acid | 0.026 |
| Tetrasodium EDTA | 0.02 |
| Trideceth-10 | 0.009 |
| *Myrica gale* oil | 3 |

### Method

EDTA and hydroxyethylcellulose were added to the water using homogenising to hydrate the polymer. The benzophenone-4 and Laureth-3 were then added and the bulk was heated to 70ºC. In a separate vessel, the cetyl alcohol was heated to 70ºC until melted and was then added to the bulk and mixed with a homogeniser until uniform. The product was cooled and the remaining materials, including the *Myrica gale* oil were then added and mixed. The product was made to weight using purified water.

### Example 29 - Spray Gel

| Ingredients | % |
|---|---|
| Phase 1 | |
| | |
| Aqua | to 100 |
| PVP/VA copolymer | 4.9 |
| Isopropyl alcohol | 2.5 |
| Propylene glycol | 2.29 |
| Glycerin | 2 |
| Panthenol | 0.375 |
| Benzophenone-4 | 0.2 |
| Sodium chloride | 0.03 |
| Wheat amino acids | 0.028 |
| *Morus alba* | 0.0046 |
| *Myrica gale* oil | 0.5 |
| | |

| Phase 2 | |
|---|---|
| PEG-40 hydrogenated castor oil | 1 |
| Parfum | 0.3 |
| | |

| Phase 3 | |
|---|---|
| | |
| Alcohol denat. | 45 |
| Dimethicone copolyol | 1 |

### Method

The materials in phase 1 were mixed until uniform using a propeller stirrer. The materials in phase 2 were pre-mixed and added to phase 1. The materials in phase 3 were mixed and added to the bulk. The product was made to weight using purified water.

### Example 30 - Dry Scalp Shampoo

| Ingredients | % |
|---|---|
| Aqua | to 100 |
| Sodium laureth sulfate | 7 |
| Sodium chloride | 2.23 |
| Cocamidopropyl betaine | 1.96 |
| Laureth-3 | 1 |
| Panthenol | 0.375 |
| Propylene glycol | 0.29 |
| Piroctone olamine | 0.25 |
| Benzophenone-4 | 0.2 |
| Phenoxyethanol | 0.162 |
| Wheat amino acids | 0.14 |
| Polyquaternium-39 | 0.1 |
| Alcohol denat. | 0.095 |
| Citric acid | 0.06 |
| Methyldibromo glutaronitrile | 0.04 |
| Tetrasodium EDTA | 0.02 |
| *Myrica gale* oil | 7 |

### Method

EDTA, citric acid, benzophenone-4 and sodium chloride were added and mixed using a propeller stirrer until all materials were dissolved and uniform. The sodium laureth sulfate and piroctone olamine were then added and stirred until homogenous. The remaining materials, including the *Myrica gale* oil were then added and the product was stirred until uniform and homogenous. The product was made to weight with purified water.

### Example 31 - Moisturising Shampoo

| Ingredients | % |
|---|---|
| Aqua | to 100 |
| Sodium laureth sulfate | 8.24 |
| Cocamidopropyl betaine | 2.8 |
| Cocamide DEA | 1.63 |
| Panthenol | 1.5 |
| Sodium chloride | 1.5 |
| Laureth-3 | 1.194 |
| Parfum | 0.5 |
| Propylene glycol | 0.5 |
| Polyquaternium-10 | 0.273 |
| Glycerin | 0.2 |
| Wheat amino acids | 0.14 |
| Alcohol denat. | 0.095 |
| Dimethicone propyl PG-betaine | 0.09 |
| Citric acid | 0.04 |
| Tetrasodium EDTA | 0.02 |
| *Myrica gale* oil | 3.8 |

### Method

EDTA and polyquaternium-1 0 were added to the water and the polymer was hydrated using a homogeniser. The citric acid, sodium chloride and benzophenone-4 were added and stirred until uniform. The remaining materials, including the *Myrica gale* oil were added individually and the product was mixed using a propeller stirrer until homogenous. The product was made to weight using purified water.

### Example 32 - Aftersun Treatment Lotion

| Ingredients | % |
|---|---|
| Aqua | to 100 |
| Hydrated silica | 5 |
| Isopropyl palmitate | 4 |
| Arachidyl propionate | 2 |
| Dimethicone | 2 |
| Glycerin | 2 |
| Steareth-21 | 1.96 |
| Steareth-2 | 1.683 |
| Cetyl alcohol | 1 |
| Tribehenin | 1 |
| Glyceryl stearate | 1 |
| Paraffinum liquidum | 0.994 |
| Panthenol | 0.75 |
| Parfum | 0.3 |
| Xanthan gum | 0.3 |
| Sodium citrate | 0.25 |
| *Myrica gale* oil | 0.2 |
| Hydroxyethylcellulose | 0.1 |
| Bisabolol | 0.095 |
| Citric acid | 0.05 |
| Preservative | q.s |
| Sodium ascorbyl phosphate | 0.15 |
| *Morus alba* | 0.0023 |
| *Panax ginseng* | 0.03 |

### Method

### Stage 1

The citric acid, sodium citrate and hydroxyethylcellulose are added to the water. Using a propellor stirrer, the mixture is stirred until dispersed. The xanthan gum is pre-dispersed in the glycerin and this is then added to the bulk, which is then heated to 70ºC.

### Stage 2

The isopropyl palmitate, arachidyl propionate, dimethicone, steareth-21, steareth-2, cetyl alcohol, tribehenin, glyceryl stearate, paraffinum liquidum are mixed and heated to 70ºC to melt the waxes.

### Stage 3

Using a homogeniser, stage 2 is added to stage 1 and is mixed until emulsified and uniform. The emulsion is cooled to below 35ºC using stirring. Once below 35ºC, the remaining materials are added, including the antioxidant complex. The product is made to weight using purified water, and mixed until uniform.

### Example 33 - Aftersun Treatment lotion

| Ingredients | % |
|---|---|
| Aqua | to 100 |
| Hydrated silica | 5 |
| Isopropyl palmitate | 4 |
| Arachidyl propionate | 2 |
| Dimethicone | 2 |
| Glycerin | 2 |
| Steareth-21 | 1.96 |
| Steareth-2 | 1.683 |
| Cetyl alcohol | 1 |
| Tribehenin | 1 |
| Glyceryl stearate | 1 |
| Paraffinum liquidum | 0.994 |
| Panthenol | 0.75 |
| Parfum | 0.3 |
| Xanthan gum | 0.3 |
| Sodium citrate | 0.25 |
| Hydroxyethylcellulose | 0.1 |
| Bisabolol | 0.095 |
| Citric acid | 0.05 |
| Preservative | q.s |
| *Myrica gale* oil | 2 |

### Method

### Stage 1

The citric acid, sodium citrate and hydroxyethylcellulose are added to the water. Using a propellor stirrer, the mixture is stirred until dispersed. The xanthan gum is pre-dispersed in the glycerin and this is then added to the bulk, which is then heated to 70ºC.

### Stage 2

The isopropyl palmitate, arachidyl propionate, dimethicone, steareth-21, steareth-2, cetyl alcohol, tribehenin, glyceryl stearate, paraffinum liquidum are mixed and heated to 70ºC to melt the waxes.

### Stage 3

Using a homogeniser, stage 2 is added to stage 1 and is mixed until emulsified and uniform. The emulsion is cooled to below 35° C using stirring.

Once below 35ºC, the *Myrica gale* oil is added. The product is made to weight using purified water, and mixed until uniform.

### Example 34 - Anti-ageing Day Cream

| Ingredients | % |
|---|---|
| Aqua | to 100 |
| Butylene glycol | 5 |
| Dicaprylyl maleate | 4 |
| Paraffinum liquidum | 4 |
| Octyl methoxycinnamate | 3 |
| Petrolatum | 3 |
| Cetyl alcohol | 2 |
| Glycerin | 2 |
| Dimethicone | 2 |
| Cetearyl alcohol | 1.6 |
| Butyl methoxydibenzoylmethane | 1 |
| Hydroxyethylcellulose | 0.4 |
| PEG-20 stearate | 0.4 |
| Polyacrylamide | 0.4 |
| Parfum | 0.3 |
| C13-14 isoparaffin | 0.215 |
| Retinyl palmitate | 0.1782 |
| Tetrasodium EDTA | 0.1 |
| Citric acid | 0.08 |
| Laureth-7 | 0.055 |
| BHT | 0.0024 |
| *Myrica gale* oil | 1.5 |
| *Morus alba* | 0.0023 |
| *Panax ginseng* | 0.03 |
| Preservative | q.s |

### Method

### Stage 1

Tetrasodium EDTA and citric acid are added to the water using a propellor stirrer. The hydroxyethylcellulose is added and dispersed using a homogeniser. butylene glycol, glycerin and methylparaben are added and the bulk is heated to 70ºC.

### Stage 2

The dicaprylyl maleate, paraffinum liquidum, octyl methoxycinnamate, petrolatum, cetyl alcohol, dimethicone, cetearyl alcohol, butyl methoxydibenzoylmethane, PEG-20 stearate, C13-14 isoparaffin, laureth-7 and BHT are mixed and heated to 70ºC to melt the waxes.

### Stage 3

Using a homogeniser, stage 2 is added to stage 1 and the bulk is mixed until emulsified and stable. The product is then cooled to below 35ºC using stirring. The remaining raw materials, including the antioxidant complex are added and the product is mixed using a propellor stirrer until uniform. The product is made to weight using purified water.

### Example 35 - Anti-ageing Day Cream

| Ingredients | % |
|---|---|
| Aqua | to 100 |
| Butylene glycol | 5 |
| Dicaprylyl maleate | 4 |
| Paraffinum liquidum | 4 |
| Octyl methoxycinnamate | 3 |
| Petrolatum | 3 |
| Cetyl Alcohol | 2 |
| Glycerin | 2 |
| Dimethicone | 2 |
| Cetearyl alcohol | 1.6 |
| Butyl methoxydibenzoylmethane | 1 |
| Hydroxyethylcellulose | 0.4 |
| PEG-20 stearate | 0.4 |
| Polyacrylamide | 0.4 |
| Parfum | 0.3 |
| C13-14 isoparaffin | 0.215 |
| Retinyl palmitate | 0.1782 |
| Tetrasodium EDTA | 0.1 |
| Citric acid | 0.08 |
| Laureth-7 | 0.055 |
| BHT | 0.0024 |
| Magnesium ascorbyl phosphate | 1.5 |
| *Myrica gale* oil | 1 |
| Preservative | q.s |

### Method

### Stage 1

Tetrasodium EDTA and citric acid are added to the water using a propellor stirrer. The hydroxyethylcellulose is added and dispersed using a homogeniser. butylene glycol, glycerin and methylparaben are added and the bulk is heated to 70ºC.

### Stage 2

The dicaprylyl maleate, paraffinum liquidum, octyl methoxycinnamate, petrolatum, cetyl alcohol, dimethicone, cetearyl alcohol, butyl methoxydibenzoylmethane, PEG-20 stearate, C13-14 isoparaffin, laureth-7 and BHT are mixed and heated to 70ºC to melt the waxes.

### Stage 3

Using a homogeniser, stage 2 is added to stage 1 and the bulk is mixed until emulsified and stable. The product is then cooled to below 35ºC using stirring. The remaining raw materials, including the antioxidant complex are added and the product is mixed using a propellor stirrer until uniform. The product is made to weight using purified water.

### Example 36 - Sun Protection Lotion SPF8

| Ingredients | % |
|---|---|
| Aqua | to 100 |
| C12-15 Alkyl Benzoate | 8 |
| Butylene glycol | 5 |
| Butyl methoxydibenzoylmethane | 2.2 |
| Dimethicone | 2 |
| Polyglyceryl-3 methylglucose distearate | 2 |
| PVP/hexadecene copolymer | 1.75 |
| Octyl methoxycinnamate | 1.7 |
| Theobroma cacao | 0.5 |
| Parfum | 0.5 |
| Tocopheryl acetate | 0.2 |
| Acrylates/vinyl isodecanoate crosspolymer | 0.15 |
| Potassium hydroxide | 0.034 |
| Tetrasodium EDTA | 0.02 |
| Preservative | q.s |
| *Myrica gale* oil | 2 |

### Method

### Stage 1

The EDTA is dispersed into the water. Using a propellor stirrer, the acrylates/vinyl isodecanoate crosspolymer are added and dispersed and hydrated. Butylene glycol is added and the aqueous phase is heated to 70ºC.

### Stage 2

The C12-15 alkyl benzoate, butyl methoxydibenzoylmethane, dimethicone, polyglyceryl-3 methylglucose distearate, PVP/hexadecene copolymer, octyl methoxycinnamate, theobroma cacao and tocopheryl acetate are mixed and heated to 70ºC to melt the waxes.

### Stage 3

Using a homogeniser, stage 2 is added to stage 1 and the bulk is mixed until emulsified and uniform. The emulsion is cooled to below 35ºC with stirring. The remaining materials, including the *Myrica gale* oil are added and mixed. The product is made to weight using purified water and stirred until uniform.

### Example 37 - Sun Protection Lotion SPF8

| Ingredients | % |
|---|---|
| Aqua | to 100 |
| C12-15 Alkyl Benzoate | 8 |
| Butylene glycol | 5 |
| Butyl methoxydibenzoylmethane | 2.2 |
| Dimethicone | 2 |
| Polyglyceryl-3 methylglucose distearate | 2 |
| PVP/hexadecene copolymer | 1.75 |
| Octyl methoxycinnamate | 1.7 |
| Theobroma cacao | 0.5 |
| Parfum | 0.5 |
| Tocopheryl acetate | 0.2 |
| Acrylates/vinyl isodecanoate crosspolymer | 0.15 |
| Potassium hydroxide | 0.034 |
| Tetrasodium EDTA | 0.02 |
| Preservative | q.s |
| Magnesium ascorbyl phosphate | 0.15 |
| *Myrica gale* oil | 0.5 |
| *Panax ginseng* | 0.03 |

### Method

### Stage 1

The EDTA is dispersed into the water. Using a propellor stirrer, the acrylates/vinyl isodecanoate crosspolymer are added and dispersed and hydrated. Butylene glycol is added and the aqueous phase is heated to 70ºC.

### Stage 2

The C12-15 alkyl benzoate, butyl methoxydibenzoylmethane, dimethicone, polyglyceryl-3 methylglucose distearate, PVP/hexadecene copolymer, octyl methoxycinnamate, theobroma cacao and tocopheryl acetate are mixed and heated to 70ºC to melt the waxes.

### Stage 3

Using a homogeniser, stage 2 is added to stage 1 and the bulk is mixed until emulsified and uniform. The emulsion is cooled to below 35ºC with stirring. The remaining materials, including the antioxidant complex are added and mixed. The product is made to weight using purified water and stirred until uniform.

### Example 38- Aftersun Treatment

| Ingredients | % |
|---|---|
| Aqua | to 100 |
| Petrolatum | 3 |
| Cetyl alcohol | 2 |
| Dimethicone | 2 |
| Glycerin | 2 |
| Ceteath-20 | 1.7 |
| Paraffinum Liquidum | 1 |
| Sodium chloride | 0.8 |
| Theobroma cacao | 0.7 |
| Glyceryl stearate | 0.5 |
| Parfum | 0.3 |
| Allantoin | 0.2 |
| Hydroxyethylcellulose | 0.1 |
| Triclosan | 0.1 |
| Citric acid | 0.02 |
| Preservative | q.s |
| Sodium ascorbyl phosphate | 0.15 |
| *Morus alba* | 0.0023 |
| *Myrica gale* oil | 1.5 |

### Method

### Stage 1

Into the water, sodium chloride and citric acid are added and dispersed. Using a propellor stirrer, hydroxyethylcellulose is added and dispersed. This phase is then heated to 70ºC.

### Stage 2

The petrolatum, cetyl alcohol, dimethicone, ceteath-20, paraffinum liquidum, theobroma cacao and glyceryl stearate are mixed and heated to 70ºC to melt the waxes.

### Stage 3

Using a homogeniser stage 2 is added to stage 1; this is mixed until emulsified and uniform. The emulsion is then cooled to below 35ºC with stirring. The remaining materials, including the antioxidant complex are then added and mixed. The product is made to weight using purified water and stirred until uniform.

### Example 39 - Aftersun Treatment

| Ingredients | % |
|---|---|
| Aqua | to 100 |
| Petrolatum | 3 |
| Cetyl alcohol | 2 |
| Dimethicone | 2 |
| Glycerin | 2 |
| Ceteath-20 | 1.7 |
| Paraffinum liquidum | 1 |
| Sodium chloride | 0.8 |
| Theobroma cacao | 0.7 |
| Glyceryl stearate | 0.5 |
| Parfum | 0.3 |
| Allantoin | 0.2 |
| Hydroxyethylcellulose | 0.1 |
| Triclosan | 0.1 |
| Citric acid | 0.02 |
| Preservative | q.s |
| *Myrica gale* oil | 5 |

### Method

### Stage 1

Into the water, sodium chloride and citric acid are added and dispersed. Using a propellor stirrer, hydroxyethylcellulose is added and dispersed. This phase is then heated to 70ºC.

### Stage 2

The petrolatum, cetyl alcohol, dimethicone, ceteath-20, paraffinum liquidum, theobroma cacao and glyceryl stearate are mixed and heated to 70ºC to melt the waxes.

### Stage 3

Using a homogeniser stage 2 is added to stage 1; this is mixed until emulsified and uniform. The emulsion is then cooled to below 35ºC with stirring. The remaining materials, including the *Myrica gale* oil are then added and mixed. The product is made to weight using purified water and stirred until uniform.

### Example 40 - Eye Contour Treatment Cream

| Ingredients | % |
|---|---|
| Aqua | to 100 |
| Butylene glycol | 6 |
| Paraffinum liquidum | 5 |
| Octyl methoxycinnamate | 4 |
| Dimethicone | 2 |
| Petrolutum | 2 |
| Cetearyl octanoate | 1.8 |
| Cetearyl alcohol | 1.6 |
| Glyceryl stearate | 1.5 |
| Cetyl alcohol | 1 |
| Prunus dulcis | 1 |
| Glycerin | 0.57 |
| Hydrogenated vegetable glycerides citrate | 0.5 |
| Tocopheryl acetate | 0.5 |
| Bisabolol | 0.475 |
| Panthenol | 0.45 |
| Sodium phosphate | 0.42 |
| PEG-20 stearate | 0.4 |
| Isopropyl myristate | 0.2 |
| Carbomer | 0.15 |
| PEG-12 isostearate | 0.125 |
| Allantoin | 0.1 |
| Tetrasodium EDTA | 0.1 |
| Lactic acid | 0.088 |
| Disodium phophate | 0.083 |
| *Myrica gale* oil | 10 |
| Preservative | q.s |

### Method

### Stage 1

Into the water, citric acid, EDTA, sodium phosphate, disodium phosphate and lactic acid are added and dispersed. Using a homogeniser, carbomer is added and hydrated. The aqueous phase is then heated to 70ºC.

### Stage 2

The paraffinum liquidum, octyl methoxycinnamate, dimethicone, petrolatum, cetearyl octanoate, cetearyl alcohol, glyceryl stearate, cetyl alcohol, hydrogenated vegetable glycerides citrate, tocopheryl acetate, PEG-20 stearate, isopropyl myristate and PEG-12 isostearate are mixed and heated to 70ºC to melt the waxes.

### Stage 3

Using a homogeniser, stage 2 is added to stage 1 and this is mixed until emulsified and uniform. The emulsion is then cooled to below 35ºC using stirring. The remaining materials, including the *Myrica gale* oil are then added and mixed. The product is then made to weight using purified water and is stirred until uniform.

### Example 41 - Eye Contour Treatment Cream

| Ingredients | % |
|---|---|
| Aqua | to 100 |
| Butylene glycol | 6 |
| Paraffinum liquidum | 5 |
| Octyl methoxycinnamate | 4 |
| Dimethicone | 2 |
| Petrolutum | 2 |
| Cetearyl octanoate | 1.8 |
| Cetearyl alcohol | 1.6 |
| Glyceryl stearate | 1.5 |
| Cetyl alcohol | 1 |
| Prunus dulcis | 1 |
| Glycerin | 0.57 |
| Hydrogenated vegetable glycerides citrate | 0.5 |
| Tocopheryl acetate | 0.5 |
| Bisabolol | 0.475 |
| Panthenol | 0.45 |
| Sodium phosphate | 0.42 |
| PEG-20 stearate | 0.4 |
| Isopropyl myristate | 0.2 |
| Carbomer | 0.15 |
| PEG-12 isostearate | 0.125 |
| Allantoin | 0.1 |
| Tetrasodium EDTA | 0.1 |
| Lactic acid | 0.088 |
| Disodium phophate | 0.083 |
| Potassium hydroxide | 0.051 |
| Magnesium ascorbyl phosphate | 1.5 |
| *Morus alba* | 0.023 |
| *Panax ginseng* | 0.03 |
| *Myrica gale* oil | 0.1 |
| Preservative | q.s |

### Method

### Stage 1

Into the water, citric acid, EDTA, sodium phosphate, disodium phosphate and lactic acid are added and dispersed. Using a homogeniser, carbomer is added and hydrated. The aqueous phase is then heated to 70ºC.

### Stage 2

The paraffinum liquidum, octyl methoxycinnamate, dimethicone, petrolatum, cetearyl octanoate, cetearyl alcohol, glyceryl stearate, cetyl alcohol, hydrogenated vegetable glycerides citrate, tocopheryl acetate, PEG-20 stearate, isopropyl myristate and PEG-12 isostearate are mixed and heated to 70ºC to melt the waxes.

### Stage 3

Using a homogeniser, stage 2 is added to stage 1 and this is mixed until emulsified and uniform. The emulsion is then cooled to below 35ºC using stirring. The remaining materials, including the antioxidant complex are then added and mixed. The product is then made to weight using purified water and is stirred until uniform.

### Example 42 - Skin Refreshing cream

| Ingredients | % |
|---|---|
| Aqua | to 100 |
| Butylene glycol | 7.5 |
| Silica | 7.2 |
| Arabinogalactan | 5.35 |
| Dimethicone | 5.35 |
| Petrolatum | 5.35 |
| Hydrated silica | 3.75 |
| Steareth-2 | 2.7 |
| Prunus dulcis | 2.7 |
| Steareth-21 | 0.9 |
| PVP/hexadecene copolymer | 0.8 |
| Carbomer | 0.32 |
| Sodium PCA | 0.2 |
| Parfum | 0.2 |
| Hydroxyethylcellulose | 0.16 |
| Potassium hydroxide | 0.1 |
| Propylene glycol | 0.1 |
| Magnesium ascorbyl phosphate | 1 |
| *Morus alba* | 0.0023 |
| *Panax ginseng* | 0.03 |
| Preservative | q.s |
| *Myrica gale* oil | 0.5 |

### Method

### Stage 1

Into the water, the carbomer is added and hydrated using a homogeniser. The aqueous phase is then heated to 70ºC.

### Stage 2

The silica, arabinogalactan, PVP/hexadecene copolymer, dimethicone, petrolatum, hydrated silica, steareth-2 and steareth-21 are mixed and heated to 70ºC to melt the waxes.

### Stage 3

Using a homogeniser, stage 2 is added to stage 1 and this is mixed until emulsified and uniform. The emulsion is then cooled to below 35ºC using stirring. The remaining materials, including the antioxidant complex are then added and mixed. The product is then made to weight using purified water and is stirred until uniform.

### Example 43 - Daily Skin Protection Lotion

| Ingredients | % |
|---|---|
| Aqua | to 100 |
| Dimethicone | 5 |
| Glycerin | 3 |
| Kaolin | 3 |
| Dicaprylyl maleate | 2.5 |
| Isopropyl myristate | 2.5 |
| Stearate-2 | 2 |
| Octyl methoxycinnamate | 1 |
| Steareth-21 | 1 |
| Cetyl alcohol | 0.75 |
| Butyl methoxydibenzoylmethane | 0.5 |
| Propylene glycol | 0.5 |
| Hydroxyethylcellulose | 0.4 |
| Xanthan gum | 0.24 |
| Serica | 0.1 |
| Sodium C8-16 isoalkylsuccinyl lactoglobulin sulfonate | 0.1 |
| Tetrasodium EDTA | 0.1 |
| Citric acid | 0.05 |
| Magnesium ascorbyl phosphate | 0.5 |
| *Morus alba* | 0.0023 |
| *Panax ginseng* | 0.03 |
| Preservative | q.s |
| *Myrica gale* oil | 0.25 |

### Method

### Stage 1

Into the water, the citric acid and EDTA are added and dispersed. The hydroxyethylcellulose is added and hydrated using a propellor stirrer. Xanthan gum is pre-dispersed in glycerin and added to the bulk. This is stirred until uniform. The aqueous phase is then heated to 70ºC.

### Stage 2

The dimethicone, dicaprylyl maleate, isopropyl myristate, stearate-2, octyl methoxycinnamate, steareth-21, cetyl alcohol and butyl methoxydibenzoylmethane are mixed and heated to 70ºC to melt the waxes.

### Stage 3

Using a homogeniser, stage 2 is added to stage 1 and this is mixed until emulsified and uniform. The emulsion is then cooled to below 35ºC using stirring. The remaining materials, including the antioxidant complex are then added and mixed. The product is then made to weight using purified water and is stirred until uniform.

### Example 44- Anti-ageing Night Cream

| Ingredients | % |
|---|---|
| Aqua | to 100 |
| Glycerin | 5 |
| Paraffinum liquidum | 4.5 |
| Dicaprylyl maleate | 3 |
| Dimethicone | 3 |
| Petrolatum | 3 |
| Paraffin | 2.9 |
| Cetyl alcohol | 2 |
| Steareth-2 | 2 |
| Glyceryl stearate | 1.5 |
| Butyrospermum parkii | 1.5 |
| Steareth-21 | 1 |
| Mannitol | 1 |
| Cera microcristallina | 0.262 |
| Buxus chinensis | 0.5 |
| Propylene glycol | 0.48 |
| Parfum | 0.4 |
| Borago officinalis | 0.3 |
| Hydroxyethylcellulose | 0.3 |
| Lactis proteinum | 0.3 |
| Xanthan gum | 0.25 |
| Alcohol denat. | 0.08 |
| Sodium citrate | 0.08 |
| Lecithin | 0.075 |
| BHT | 0.05 |
| Faex | 0.04 |
| Phospholipids | 0.03 |
| Citric acid | 0.025 |
| *Myrica gale* oil | 1.07 |
| *Panax ginseng* | 0.03 |
| Preservative | q.s |

### Method

### Stage 1

Into the water, the citric acid and sodium citrate are added and dispersed. The hydroxyethylcellulose is added and hydrated using a propellor stirrer. Xanthan gum is pre-dispersed in glycerin and added to the bulk. This is stirred until uniform. The aqueous phase is then heated to 70ºC.

### Stage 2

The paraffinum liquidum, dicaprylyl maleate, dimethicone, petrolatum, paraffin, cetyl alcohol, steareth-2 , glyceryl stearate, steareth-21, cera microcristallina and BHT are mixed and heated to 70ºC to melt the waxes.

### Stage 3

Using a homogeniser, stage 2 is added to stage 1 and this is mixed until emulsified and uniform. The emulsion is then cooled to below 35ºC using stirring. The remaining materials, including the antioxidant complex are then added and mixed. The product is then made to weight using purified water and is stirred until uniform.

### Example 45 - Sun protection Lotion for Sensitive Skin - SPF15

| Ingredients | % |
|---|---|
| Aqua | to 100 |
| C12-15 alkyl benzoate | 12 |
| Butylene glycol | 5 |
| Octyl methoxycinnamate | 3.8 |
| Butyl methoxydibenzoylmethane | 3 |
| Dimethicone | 2 |
| Polyglyceryl-3 methylglucose distearate | 2 |
| PVP/hexadecene copolymer | 1.75 |
| C18-36 acid glycol ester | 1.5 |
| Polysorbate 60 | 0.5 |
| Titanium dioxide | 0.3 |
| Tocopheryl acetate | 0.2 |
| Acrylates/vinyl isodecanoate crosspolymer | 0.14 |
| Potassium hydroxide | 0.035 |
| Tetrasodium EDTA | 0.02 |
| Preservative | q.s |
| *Myrica gale* oil | 0.05 |
| *Morus alba* | 0.0023 |
| *Panax ginseng* | 0.03 |

### Method

### Stage 1

Into the water, citric acid is added and dispersed. The acrlyates/vinyl isodecanoate crosspolymer are added and dispersed using a propellor stirrer. The aqueous phase is then heated to 70ºC.

### Stage 2

The C12-15 alkyl benzoate, PVP/hexadecene copolymer, octyl methoxycinnamate, butyl methoxydibenzoylmethane, dimethicone, polyglyceryl-3 methylglucose distearate, C18-36 acid glycol ester, polysorbate 60, titanium dioxide and tocopheryl acetate are mixed and heated to 70ºC to melt the waxes.

### Stage 3

Using a homogeniser, stage 2 is added to stage 1 and this is mixed until emulsified and uniform. The emulsion is then cooled to below 35ºC using stirring. The remaining materials, including the antioxidant complex are then added and mixed. The product is then made to weight using purified water and is stirred until uniform.

### Example 46 - Sun protection Lotion for Sensitive Skin - SPF15

| Ingredients | % |
|---|---|
| Aqua | to 100 |
| C12-15 alkyl benzoate | 12 |
| Butylene glycol | 5 |
| Octyl methoxycinnamate | 3.8 |
| Butyl methoxydibenzoylmethane | 3 |
| Dimethicone | 2 |
| Polyglyceryl-3 methylglucose distearate | 2 |
| PVP/hexadecene copolymer | 1.75 |
| C18-36 acid glycol ester | 1.5 |
| Polysorbate 60 | 0.5 |
| Titanium dioxide | 0.3 |
| Tocopheryl acetate | 0.2 |
| Acrylates/vinyl isodecanoate crosspolymer | 0.14 |
| Potassium hydroxide | 0.035 |
| Tetrasodium EDTA | 0.02 |
| Preservative | q.s |
| Magnesium ascorbyl phosphate | 0.05 |
| *Morus alba* | 0.0023 |
| *Panax ginseng* | 0.03 |
| *Myrica gale* oil | 0.1 |

### Method

### Stage 1

Into the water, citric acid is added and dispersed. The acrlyates/vinyl isodecanoate crosspolymer are added and dispersed using a propellor stirrer. The aqueous phase is then heated to 70ºC.

### Stage 2

The C12-15 alkyl benzoate, PVP/hexadecene copolymer, octyl methoxycinnamate, butyl methoxydibenzoylmethane, dimethicone, polyglyceryl-3 methylglucose distearate, C18-36 acid glycol ester, polysorbate 60, titanium dioxide and tocopheryl acetate are heated to 70ºC to melt the waxes.

### Stage 3

Using a homogeniser, stage 2 is added to stage 1 and this is mixed until emulsified and uniform. The emulsion is then cooled to below 35ºC using stirring. The remaining materials, including the antioxidant complex are then added and mixed. The product is then made to weight using purified water and is stirred until uniform.

### Example 47 - Sun Protection Cream For Sensitive Skin

| Ingredients | % |
|---|---|
| Aqua | to 100 |
| Octyl stearate | 13.5 |
| Zinc oxide | 13.5 |
| Isopropyl myristate | 5 |
| Butylene glycol | 3 |
| Isohexadecane | 3 |
| Titanium dioxide | 2 |
| Polyglyceryl-3 oleate | 1.75 |
| Cetyl dimethicone copolyol | 1.35 |
| Magnesium sulfate | 0.75 |
| Sodium chloride | 0.75 |
| Aluminium stearate | 0.18 |
| Alumina | 0.15 |
| Lecithin | 0.13 |
| Isopropyl palmitate | 0.05 |
| Sodium ascorbyl phosphate | 0.10 |
| *Morus alba* | 0.0023 |
| *Myrica gale* oil | 1 |

### Method

### Stage 1

Into the water, magnesium sulfate, sodium chloride and butylene glycol are added and dispersed. The aqueous phase is then heated to 70ºC.

### Stage 2

The octyl stearate, isopropyl myristate, isohexadecane, titanium dioxide, polyglyceryl-3 oleate, cetyl dimethicone copolyol, aluminium stearate, lecithin and isopropyl palmitate are mixed and heated to 70ºC to melt the waxes.

### Stage 3

Using a propellor stirrer, stage 2 is added to stage 1. Once uniform, the emulsion is transferred to a homogeniser and mixed to generate the viscosity. The emulsion is then cooled to below 35ºC using stirring. The remaining materials, including the antioxidant complex are then added and mixed. The product is then made to weight using purified water and is stirred until uniform.

### Example 48 - Sun Protection Cream For Sensitive Skin

| Ingredients | % |
|---|---|
| Aqua | to 100 |
| Octyl stearate | 13.5 |
| Zinc oxide | 13.5 |
| Isopropyl myristate | 5 |
| Butylene glycol | 3 |
| Isohexadecane | 3 |
| Titanium dioxide | 2 |
| Polyglyceryl-3 oleate | 1.75 |
| Cetyl dimethicone copolyol | 1.35 |
| Magnesium sulfate | 0.75 |
| Sodium chloride | 0.75 |
| Aluminium stearate | 0.18 |
| Alumina | 0.15 |
| Lecithin | 0.13 |
| Isopropyl palmitate | 0.05 |
| Magnesium ascorbyl phosphate | 0.15 |
| *Myrica gale* oil | 3 |
| Panax ginseng | 0.03 |

### Method

### Stage 1

Into the water, magnesium sulfate, sodium chloride and butylene glycol are added and dispersed. The aqueous phase is then heated to 70ºC.

### Stage 2

The octyl stearate, isopropyl myristate, isohexadecane, titanium dioxide, polyglyceryl-3 oleate, cetyl dimethicone copolyol, aluminium stearate, lecithin and isopropyl palmitate are mixed and heated to 70ºC to melt the waxes.

### Stage 3

Using a propellor stirrer, stage 2 is added to stage 1. Once uniform, the emulsion is transferred to a homogeniser and mixed to generate the viscosity. The emulsion is then cooled to below 35ºC using stirring. The remaining materials, including the antioxidant complex are then added and mixed. The product is then made to weight using purified water and is stirred until uniform.

### Example 49 - Anti-ageing Foundation

| Ingredients | % |
|---|---|
| Aqua | to 100 |
| Butylene glycol | 9.8 |
| Cetearyl isononanoate | 4.9 |
| Dimethicone | 3.2 |
| Glycerin | 1.96 |
| Silica | 1.9 |
| Caprylic/capric triglyceride | 1.67 |
| Paraffinum liquidum | 1.67 |
| Petrolatum | 1.67 |
| Hydrogenated coco-glycerides | 1.67 |
| Cetearyl octanoate | 1.5 |
| Cetearyl alcohol | 1.35 |
| Octyl methoxycinnamate | 1.28 |
| Talc | 1 |
| Glyceryl stearate | 0.95 |
| PEG-100 stearate | 0.9 |
| Butyl methoxydibenzoylmethane | 0.6 |
| Saccharide isomerate | 0.54 |
| Lactic acid | 0.45 |
| Sodium polyacrylate | 0.45 |
| Boron nitride | 0.42 |
| Sodium PCA | 0.4 |
| Borago officinalis | 0.4 |
| Tocopheryl acetate | 0.4 |
| PVP/hexadecene copolymer | 0.4 |
| PEG-20 stearate | 0.33 |
| Glycolic acid | 0.2 |
| Sodium stearoyl lactylate | 0.2 |
| Isopropyl myristate | 0.17 |
| Polyaminopropyl biguanide | 0.16 |
| Tetrasodium EDTA | 0.1 |
| Xanthan gum | 0.1 |
| Citric acid | 0.06 |
| Alcohol denat. | 0.04 |
| Lecithin | 0.037 |
| Preservative | q.s |
| *Rosmarinus officinalis* | 0.1 |
| *Myrica gale* oil | 3.9 |

### Method

### Stage 1

Into the water, citric acid, EDTA and lactic acid are added and dispersed. Xanthan gum is pre-dispersed in butylene glycol and is added to the bulk. The aqueous phase is then heated to 70ºC.

### Stage 2

The cetearyl isononanoate, dimethicone, silica, PVP/hexadecene copolymer, caprylic/capric triglyceride, paraffinum liquidum, petrolatum, hydrogenated coco-glycerides, cetearyl octanoate, cetearyl alcohol, octyl methoxycinnamate, talc, glyceryl stearate, PEG-100 stearate, butyl methoxydibenzoylmethane, borago officinalis, tocopheryl acetate, sodium stearoyl lactylate, isopropyl myristate and lecithinoil phase are mixed and heated to 70ºC to melt the waxes.

### Stage 3

Using a homogeniser, stage 2 is added to stage 1 and this is mixed until emulsified and uniform. The emulsion is then cooled to below 35ºC using stirring.

The remaining materials, including the antioxidant complex are then added and mixed. The product is then made to weight using purified water and is stirred until uniform.

### Example 50 - Anti-ageing Foundation

| Ingredients | % |
|---|---|
| Aqua | to 100 |
| Butylene glycol | 9.8 |
| Cetearyl isononanoate | 4.9 |
| Dimethicone | 3.2 |
| Glycerin | 1.96 |
| Silica | 1.9 |
| Caprylic/capric triglyceride | 1.67 |
| Paraffinum liquidum | 1.67 |
| Petrolatum | 1.67 |
| Hydrogenated coco-glycerides | 1.67 |
| Cetearyl octanoate | 1.5 |
| Cetearyl alcohol | 1.35 |
| Octyl methoxycinnamate | 1.28 |
| Talc | 1 |
| Glyceryl stearate | 0.95 |
| PEG-100 stearate | 0.9 |
| Butyl methoxydibenzoylmethane | 0.6 |
| Saccharide isomerate | 0.54 |
| Lactic acid | 0.45 |
| Sodium polyacrylate | 0.45 |
| Boron nitride | 0.42 |
| Sodium PCA | 0.4 |
| Borago officinalis | 0.4 |
| Tocopheryl acetate | 0.4 |
| PVP/hexadecene copolymer | 0.4 |
| PEG-20 stearate | 0.33 |
| Glycolic acid | 0.2 |
| Sodium stearoyl lactylate | 0.2 |
| Isopropyl myristate | 0.17 |
| Polyaminopropyl biguanide | 0.16 |
| Tetrasodium EDTA | 0.1 |
| Xanthan gum | 0.1 |
| Citric acid | 0.06 |
| Alcohol denat. | 0.04 |
| Lecithin | 0.037 |
| Preservative | q.s |
| *Origanum vulgare* | 0.1 |
| *Morus alba* | 0.0023 |
| *Panax ginseng* | 0.03 |
| *Myrica gale* oil | 1 |

### Method

### Stage 1

Into the water, citric acid, EDTA and Lactic acid are added and dispersed. Xanthan gum is pre-dispersed in butylene glycol and is added to the bulk. The aqueous phase is then heated to 70ºC.

### Stage 2

The cetearyl isononanoate, dimethicone, Silica, PVP/hexadecene copolymer, caprylic/capric triglyceride, paraffinum liquidum, petrolatum, hydrogenated coco-glycerides, cetearyl octanoate, cetearyl alcohol, octyl methoxycinnamate, talc, glyceryl stearate, PEG-100 stearate, butyl methoxydibenzoylmethane, borago officinalis, tocopheryl acetate, sodium stearoyl lactylate, isopropyl myristate and lecithinoil phase are mixed and heated to 70ºC to melt the waxes.

### Stage 3

Using a homogeniser, stage 2 is added to stage 1 and this is mixed until emulsified and uniform. The emulsion is then cooled to below 35ºC using stirring. The remaining materials, including the antioxidant complex are then added and mixed. The product is then made to weight using purified water and is stirred until uniform.

### Example 51 - Sun Protection Spray - SPF15

| Ingredients | % |
|---|---|
| Aqua | to 100 |
| Dicaprylyl maleate | 12 |
| Butylene glycol | 5 |
| Octyl methoxycinnamate | 4 |
| Butyl methoxydibenzoylmethane | 3.5 |
| Dimethicone | 3 |
| Polyglyceryl-3 methylglucose distearate | 3 |
| Acrylates/octylacrylamide copolymer | 2 |
| C18-36 acid glycol ester | 1.5 |
| Triethanolamine | 0.5 |
| Tocopheryl acetate | 0.2 |
| Acrylates/vinyl isodecanoate crosspolymer | 0.05 |
| Tetrasodium EDTA | 0.02 |
| Potassium hydroxide | 0.015 |
| Preservative | q.s |
| *Myrica gale* oil | 2 |

### Method

### Stage 1

Into the water, EDTA is added and dispersed. Acrylates/vinyl isodecanoate crosspolymer are added and dispersed using a propellor stirrer. Butylene glycol is added and dispersed. The aqueous phase is then heated to 70ºC.

### Stage 2

The dicaprylyl maleate, Acrylates/octylacrylamide copolymer, octyl methoxycinnamate, butyl methoxydibenzoylmethane, dimethicone, polyglyceryl-3 methylglucose, C18-36 acid glycol ester and tocopheryl acetate are mixed and heated to 80ºC to melt the waxes.

### Stage 3

Using a homogeniser, stage 2 is added to stage 1 and this is mixed until emulsified and uniform. The emulsion is then cooled to below 35ºC using stirring. The remaining materials, including the *Myrica gale* oil are then added and mixed. The product is then made to weight using purified water and is stirred until uniform.

### Example 52 - Sun Protection Spray - SPF15

| Ingredients | % |
|---|---|
| Aqua | to 100 |
| Dicaprylyl maleate | 12 |
| Butylene glycol | 5 |
| Octyl methoxycinnamate | 4 |
| Butyl methoxydibenzoylmethane | 3.5 |
| Dimethicone | 3 |
| Polyglyceryl-3 methylglucose distearate | 3 |
| Acrylates/octylacrylamide copolymer | 2 |
| C18-36 acid glycol ester | 1.5 |
| Triethanolamine | 0.5 |
| Tocopheryl acetate | 0.2 |
| Acrylates/vinyl isodecanoate crosspolymer | 0.05 |
| Tetrasodium EDTA | 0.02 |
| Potassium hydroxide | 0.015 |
| Preservative | q.s |
| *Myrica gale* oil | 0.3 |
| *Panax ginseng* | 0.03 |

### Method

### Stage 1

Into the water, EDTA is added and dispersed. Acrylates/vinyl isodecanoate crosspolymer are added and dispersed using a propellor stirrer. Butylene glycol is added and dispersed. The aqueous phase is then heated to 70ºC.

### Stage 2

The dicaprylyl maleate, Acrylates/octylacrylamide copolymer, octyl methoxycinnamate, butyl methoxydibenzoylmethane, dimethicone, polyglyceryl-3 methylglucose, C18-36 acid glycol ester and tocopheryl acetate are mixed and heated to 70ºC to melt the waxes.

### Stage 3

Using a homogeniser, stage 2 is added to stage 1 and this is mixed until emulsified and uniform. The emulsion is then cooled to below 35ºC using stirring. The remaining materials, including the antioxidant complex are then added and mixed. The product is then made to weight using purified water and is stirred until uniform.

### Example 53 - Toner & Cleanser 2 In 1

| Ingredients | % |
|---|---|
| Alcohol denat. | 48 |
| Aqua | to 100 |
| PEG-8 | 6 |
| Glycerin | 2 |
| Propylene glycol | 0.5 |
| Sodium C8-16 isoalkylsuccinyl lactoglobulin sulfonate | 0.02 |
| *Laminaria saccharina* | 0.01 |
| *Hamamelis virginiana* | 0.006 |
| *Citrullus vulgaris* | 0.001 |
| Preservative | q.s |
| Sodium ascorbyl phosphate | 1.5 |
| *Morus alba* | 0.0023 |
| *Myrica gale* oil | 0.5 |

### Method

Into the water, alcohol denat. is added and dispersed until uniform. Using a propellor stirrer, all materials including the antioxidant complex, are slowly added and stirred until uniform. The product is made to weight using purified water and stirred until uniform.

### Example 54 - Toner & Cleanser 2 In 1

| Ingredients | % |
|---|---|
| Alcohol denat. | 48 |
| Aqua | to 100 |
| PEG-8 | 6 |
| Glycerin | 2 |
| Propylene glycol | 0.5 |
| Sodium C8-16 isoalkylsuccinyl lactoglobulin sulfonate | 0.02 |
| *Laminaria saccharina* | 0.01 |
| *Hamamelis virginiana* | 0.006 |
| *Citrullus vulgaris* | 0.001 |
| Preservative | q.s |
| Magnesium ascorbyl phosphate | 1.5 |
| *Morus alba* | 0.0023 |
| *Panax ginseng* | 0.03 |
| *Myrica gale oil* | 1.5 |

### Method

Into the water, alcohol denat. Is added and dispersed until uniform. Using a propellor stirrer, all materials including the antioxidant complex, are slowly added and stirred until uniform. The product is made to weight using purified water and stirred until uniform.

### Example 55 - Skin pH Balancing Toner

| Ingredients | % |
|---|---|
| Aqua | to 100 |
| Alcohol denat. | 7.9 |
| Butylene glycol | 2 |
| Dimethicone copolyol | 1.5 |
| Sodium lactate | 0.6 |
| Glycerin | 0.5 |
| Allantoin | 0.1 |
| Propylene glycol | 0.1 |
| Lactic acid | 0.002 |
| Preservative | q.s |
| *Myrica gale* oil | 2 |

### Method

Into the water, lactic acid and alcohol denat are separately added and dispersed until uniform. Using a propellor stirrer, all materials including the *Myrica gale* oil, are slowly added and stirred until uniform. The product is made to weight using purified water and stirred until uniform.

### Example 56 - Skin pH Balancing Toner

| Ingredients | % |
|---|---|
| Aqua | to 100 |
| Alcohol denat. | 7.9 |
| Butylene glycol | 2 |
| Dimethicone copolyol | 1.5 |
| Sodium lactate | 0.6 |
| Glycerin | 0.5 |
| Allantoin | 0.1 |
| Propylene glycol | 0.1 |
| Lactic acid | 0.002 |
| Preservative | q.s |
| Magnesium ascorbyl phosphate | 1.5 |
| *Myrica gale* oil | 0.5 |

### Method

Into the water, lactic acid and alcohol denat are separately added and dispersed until uniform. Using a propellor stirrer, all materials including the antioxidant complex, are slowly added and stirred until uniform. The product is made to weight using purified water and stirred until uniform.

### Example 57 - pH Balanced Cleansing Lotion

| Ingredients | % |
|---|---|
| Aqua | to 100 |
| Paraffinum liquidum | 14 |
| Isopropyl palmitate | 7 |
| Glyceryl stearate | 2.5 |
| PEG-100 stearate | 2.5 |
| Butylene glycol | 2 |
| Hydrogenated vegetable glycerides citrate | 2 |
| Polysorbate 60 | 0.5 |
| Sorbitan stearate | 0.5 |
| *Persea gratissima* | 0.3 |
| *Prunus persica* | 0.3 |
| Propylene glycol | 0.3 |
| Acrylates/C10-30 alkyl acrylate crosspolymer | 0.12 |
| Potassium hydroxide | 0.05 |
| Tetrasodium EDTA | 0.02 |
| Medicago sativa | 0.0045 |
| Preservative | q.s |
| Sodium ascorbyl phosphate | 1.5 |
| *Morus alba* | 0.023 |
| *Panax ginseng* | 0.03 |
| *Myrica gale* oil | 0.4 |

### Method

### Stage 1

Into the water, EDTA is added and dispersed. Butylene glycol is then added and dispersed. The aqueous phase is then heated to 70ºC.

### Stage 2

The paraffinum liquidum, isopropyl palmitate, glyceryl stearate, PEG-100 stearate, hydrogenated vegetable glycerides citrate, polysorbate 60 and sorbitan stearate are mixed and heated to 70ºC to melt the waxes.

### Stage 3

Using a homogeniser, stage 2 is added to stage 1 and this is mixed until emulsified and uniform. The emulsion is then cooled to below 35ºC using stirring. The remaining materials, including the antioxidant complex are then added and mixed. The product is then made to weight using purified water and is stirred until uniform.

### Example 58 - pH Balanced Cleansing Lotion

| Ingredients | % |
|---|---|
| Aqua | to 100 |
| Paraffinum liquidum | 14 |
| Isopropyl palmitate | 7 |
| Glyceryl stearate | 2.5 |
| PEG-100 stearate | 2.5 |
| Butylene glycol | 2 |
| Hydrogenated vegetable glycerides citrate | 2 |
| Polysorbate 60 | 0.5 |
| Sorbitan stearate | 0.5 |
| *Persea gratissima* | 0.3 |
| *Prunus persica* | 0.3 |
| Propylene glycol | 0.3 |
| Acrylates/C10-30 alkyl acrylate crosspolymer | 0.12 |
| Potassium hydroxide | 0.05 |
| Tetrasodium EDTA | 0.02 |
| Medicago sativa | 0.0045 |
| Preservative | q.s |
| *Myrica gale* oil | 2.5 |

### Method

### Stage 1

Into the water, EDTA is added and dispersed. Butylene glycol is then added and dispersed. The aqueous phase is then heated to 70ºC.

### Stage 2

The paraffinum liquidum, isopropyl palmitate, glyceryl stearate, PEG-100 stearate, hydrogenated vegetable glycerides citrate, polysorbate 60 and sorbitan stearate are mixed and heated to 70ºC to melt the waxes.

### Stage 3

Using a homogeniser, stage 2 is added to stage 1 and this is mixed until emulsified and uniform. The emulsion is then cooled to below 35ºC using stirring.

The remaining materials, including the *Myrica gale* oil are then added and mixed. The product is then made to weight using purified water and is stirred until uniform.

### Example 59 - Lipstick

| Ingredients | % |
|---|---|
| Ricinus communis | 20 |
| Octyldodecanol | 15 |
| Pentaerythrityl tetracaprylate/caprate | 14 |
| Mica | 10 |
| Bis-diglyceryl caprylate/caprate/isostearate/ | |
| Stearate/hydroxystearate adipate | 7.5 |
| Paraffin | 5 |
| Cera microcristallina | 5 |
| Propylene glycol | 2 |
| Hydrogenated castor oil | 2 |
| Candelilla cera | 1 |
| Carnauba | 1 |
| Synthetic wax | 1 |
| Butyrospermum parkii | 1 |
| Titanium dioxide | 0.5 |
| Tocopheryl acetate | 0.2 |
| Polyquaternium-37 | 0.2 |
| Red colour | q.s |
| Magnesium ascorbyl phosphate | 1.5 |
| *Morus alba* | 0.0023 |
| *Panax ginseng* | 0.03 |
| *Myrica gale* oil | 0.1 |

### Method

### Stage 1

The antioxidant complex is pre-dispersed in propylene glycol, with stirring.

### Stage 2

The remaining materials are mixed in a vessel and heated to 85ºC until melted and uniform. The product is cooled and the antioxidant complex pre-mix is added below 70ºC. The product poured into a suitable container and allowed to cool to room temperature to set.

### Example 60 - Lipstick

| Ingredients | % |
|---|---|
| Ricinus communis | 20 |
| Octyldodecanol | 15 |
| Pentaerythrityl tetracaprylate/caprate | 14 |
| Mica | 10 |
| Bis-diglyceryl caprylate/caprate/isostearate/ | |
| Stearate/hydroxystearate adipate | 7.5 |
| Paraffin | 5 |
| Cera microcristallina | 5 |
| Propylene glycol | 2 |
| Hydrogenated castor oil | 2 |
| Candelilla cera | 1 |
| Carnauba | 1 |
| Synthetic wax | 1 |
| Butyrospermum parkii | 1 |
| Titanium dioxide | 0.5 |
| Tocopheryl acetate | 0.2 |
| Polyquaternium-37 | 0.2 |
| Red colour | q.s |
| Sodium ascorbyl phosphate | 1 |
| *Morus alba* | 0.0023 |
| *Panax ginseng* | 0.03 |
| *Sweet gale oil* | 1 |

### Method

### Stage 1

The antioxidant complex is pre-dispersed in propylene glycol, with stirring.

### Stage 2

The remaining materials are mixed in a vessel and heated to 85ºC until melted and uniform. The product is cooled and the antioxidant complex pre-mix is added below 70ºC. The product poured into a suitable container and allowed to cool to room temperature to set.

### Example 61 - Shampoo Base A

| Ingredients | | % |
|---|---|---|
| 1 | Cocamide MEA | 3.5 |
| 2 | Glycol distearate | 3.5 |
| 3 | Sodium gluceptate | 0.25 |
| 4 | Sodium metabisulfite | 2.0 |
| 5 | Sodium erthroborate | 0.3 |
| 6 | Cocamidopropyl betaine | 11.25 |
| 7 | Dilute sodium lauryl-ether sulphate | 25.75 |
| 8 | Laureth-3 | 10 |
| 9 | Oleic acid | 4 |
| 10 | Sodium silicate | 5 |
| 11 | Citric acid monohydrate | 0.1 or 1 |
| 12 | Hair dye | qs |
| 13 | *Myrica gale* oil | 2 |
| 14 | Purified water | to 100% |

### Method

1. Heat 1, 2 & 8 to 70-75ºC, until melted.
2. Disperse 3, 4, 5 & 12 into 13 and heat to 70-75ºC.
3. Stir in 6 & 7 to the water phase and maintain at 70-75ºC.
4. Add the oil phase to the water phase and homogenise for 10 minutes.
5. Cool to below 35ºC.
6. Add 9 & 10 to the mixture and stir.
7. Stir in 11 (previously dispersed in water) and 12.

### Example 62 - Shampoo Base B

| Ingredients | | % |
|---|---|---|
| 1 | Cocamide MEA | 3.5 |
| 2 | Glycol distearate | 3.5 |
| 3 | Sodium gluceptate | 0.25 |
| 4 | Sodium metabisulfite | 2.0 |
| 5 | Sodium erthroborate | 0.3 |
| 6 | Cocamidopropyl betaine | 11.25 |
| 7 | Dilute sodium lauryl-ether sulphate | 25.75 |
| 8 | Laureth-3 | 10 |
| 9 | Oleic acid | 4 |
| 10 | Sodium silicate | 7.5 |
| 11 | Citric acid monohydrate | 0.1 or 1 |
| 12 | Hair dye | qs |
| 13 | *Myrica gale* oil | 0.3 |
| 14 | Purified water | to 100% |

### Method

Prepared by the same method as Example 61.

### Example 63 - Shampoo Base C

| Ingredients | | % |
|---|---|---|
| 1 | Cocamide MEA | 3.5 |
| 2 | Glycol distearate | 3.5 |
| 3 | Sodium gluceptate | 0.25 |
| 4 | Sodium metabisulfite | 2.0 |
| 5 | Sodium erthroborate | 0.3 |
| 6 | Cocamidopropyl betaine | 11.25 |
| 7 | Dilute sodium lauryl-ether sulphate | 25.75 |
| 8 | Laureth-3 | 10 |
| 9 | Oleic acid | 4 |
| 10 | Sodium silicate | 10 |
| 11 | Citric acid monohydrate | 0.1 or 1 |
| 12 | Hair dye | qs |
| 13 | *Myrica gale* oil | 2.5 |
| 14 | Purified water | to 100% |

### Method

Prepared by the same method as Example 61.

### Example 64 - Shampoo Base D (With Ammonia)

| Ingredients | | % |
|---|---|---|
| 1 | Cocamide MEA | 3.5 |
| 2 | Glycol distearate | 3.5 |
| 3 | Sodium gluceptate | 0.25 |
| 4 | Sodium metabisulfite | 2.0 |
| 5 | Sodium erthroborate | 0.3 |
| 6 | Cocamidopropyl betaine | 11.25 |
| 7 | Dilute sodium lauryl-ether sulphate | 25.75 |
| 8 | Laureth-3 | 10 |
| 9 | Oleic acid | 4 |
| 10 | Ammonia | 5 |
| 11 | Citric acid monohydrate | 0.1 or 1 |
| 12 | Hair dye | qs |
| 13 | *Myrica gale* oil | 5 |
| 14 | Purified water | to 100% |

### Method

Prepared by the same method as Example 61, substituting ammonia for sodium silicate.

### Example 65 - Shampoo Base E (With Ammonia)

| Ingredients | | % |
|---|---|---|
| 1 | Cocamide MEA | 3.5 |
| 2 | Glycol distearate | 3.5 |
| 3 | Sodium gluceptate | 0.25 |
| 4 | Sodium metabisulfite | 2.0 |
| 5 | Sodium erthroborate | 0.3 |
| 6 | Cocamidopropyl betaine | 11.25 |
| 7 | Dilute sodium lauryl-ether sulphate | 25.75 |
| 8 | Laureth-3 | 10 |
| 9 | Oleic acid | 4 |
| 10 | Ammonia | 10 |
| 11 | Citric acid monohydrate | 0.1 or 1 |
| 12 | Hair dye | qs |
| 13 | *Myrica gale* oil | 2 |
| 14 | Purified water | to 100% |

### Method

Prepared by the same method as Example 61, substituting ammonia for sodium silicate.

### Example 66 - Conditioner Base A

| Ingredients | | % |
|---|---|---|
| 1 | Tetra sodium EDTA | 0.14 |
| 2 | Sodium benzoate | 0.1 |
| 3 | Phenoxyethanol | 0.1 |
| 4 | Citric acid monohydrate | 1.5 |
| 5 | Ceteath-25 | 1.5 |
| 6 | Cetearyl alcohol | 2.25 |
| 7 | Stearyl alcohol | 2.25 |
| 8 | Hair dyes | qs |
| 9 | Sodium silicate | 5 |
| 10 | *Myrica gale* oil | 0.4 |
| 11 | Purified water | to 100% |

### Method

1. Heat 5, 6 & 7 to 70-75ºC.
2. Heat 10 to 70-75ºC and add 9 and stir.
3. Disperse 1, 2, 4 & 8 into the water phase.
4. Add oils to water phase and homogenise for 10 minutes.
5. Cool to below 35ºC.
6. Stir in 3 and 10.

### Example 67 - Conditioner Base B

| Ingredients | | % |
|---|---|---|
| 1 | Tetra sodium EDTA | 0.14 |
| 2 | Sodium benzoate | 0.1 |
| 3 | Phenoxyethanol | 0.1 |
| 4 | Citric acid monohydrate | 1.5 |
| 5 | Ceteath-25 | 1.5 |
| 6 | Cetearyl alcohol | 2.25 |
| 7 | Stearyl alcohol | 2.25 |
| 8 | Hair dyes | qs |
| 9 | Sodium silicate | 7.5 |
| 10 | *Myrica gale* oil | 3 |
| 11 | Purified water | to 100% |

### Method

Prepared by the same method as Example 66.

### Example 68 - Conditioner Base C

| Ingredients | | % |
|---|---|---|
| 1 | Tetra sodium EDTA | 0.14 |
| 2 | Sodium Benzoate | 0.1 |
| 3 | Phenoxyethanol | 0.1 |
| 4 | Citric acid monohydrate | 1.5 |
| 5 | Ceteath-25 | 1.5 |
| 6 | Cetearyl alcohol | 2.25 |
| 7 | Stearyl alcohol | 2.25 |
| 8 | Hair dyes | qs |
| 9 | Sodium silicate | 10 |
| 10 | *Myrica gale* oil | 7 |
| 11 | Purified water | to 100% |

### Method

Prepared by the same method as Example 66.

### Example 69 - Developing Lotion

| Ingredients | | % |
|---|---|---|
| 1 | Tetra sodium EDTA | 0.1 |
| 2 | Phosphoric acid | 0.09 |
| 3 | Hydrogen peroxide | 8.75 |
| 4 | Ceteath-20 | 2.0 |
| 5 | Cetearyl alcohol | 4.0 |
| 6 | Sodium stannate | 0.005 |
| *7* | *Myrica gale* oil | 2 |
| 8 | Purified water | to 100% |

### Method

1. Disperse 1, 2 & 6 into 7. Heat to 70-75ºC.
2. Heat 4 & 5 to 70-75ºC until melted.
3. Add oil phase to the water phase and homogenise for 10 minutes.
4. Cool to below 35ºC.
5. Stir in 3 and 7.

In use, a shampoo or conditioner base (for example the formulations of Examples 61 to 63 or Examples 64 to 66) carries the hair dye molecules and is used in conjunction with a developing lotion (for example the formulation of Example 67) which delivers the oxidising agent. In Example 67 the oxidising agent is hydrogen peroxide, but other oxidising agents may be used. In a typical case, equal volumes of the base formulation and the developing lotion are mixed together, to oxidatively activate the dye molecules to react with each other. The mixture is then applied to dry hair for a period of 30 minutes. The hair is then rinsed with water and a conditioner is applied. This is followed by drying of the hair, naturally, by towel or the use of a hair dryer.

### Test Results

### A. Non-irritancy of Myrica Gale

A 96 hour patch test was carried out to assess the irritancy or otherwise of *Myrica gale* oil when applied to the skin. For comparison, a solution of sodium dodecyl sulphate and white soft paraffin were also investigated.

The results are shown in Table 1.

**Table 1**

| Test Material | Mean Erythema (E) | Mean Dryness (D) | Mean Irritation (E+D) |
|---|---|---|---|
| Sodium dodecyl sulphate (SDS) 0.3% w/v in sterile water | 4.54 | 0.72 | 5.26 |
| White soft paraffin BP 100% | 0.06 | 0.00 | 0.06 |
| *Myrica gale* oil 1% in white soft paraffin | 0.08 | 0.00 | 0.08 |
| *Myrica gale* oil 2% in white soft paraffin | 0.08 | 0.00 | 0.08 |
| *Myrica gale* oil 5% in white soft paraffin | 0.05 | 0.00 | 0.05 |
| *Myrica gale* oil 7% in white soft paraffin | 0.02 | 0.00 | 0.02 |
| *Myrica gale* oil 10% in white soft paraffin | 0.05 | 0.00 | 0.05 |

The results show that no significant adverse response was observed, even at concentrations of *Myrica gale* up to 10%.

### B. Anti-microbial Properties

The *in vitro* biostatic activity of *Myrica gale* was assessed to determine whether it has antimicrobial properties. The Minimum Inhibitory Concentration (MIC) is the lowest dilution required to inhibit growth of the test organism. The method involves exposing a range of test organisms to dilutions of the active in the agar. Following incubation, the growth of the test organisms is visually examined and compared to a control containing no active. The MIC of each material for each test organism is the lowest concentration that has been shown to inhibit growth and can be used as a guide to the effectiveness of the active. The following test organisms were used:
*1. Staphylococcus aureus* NCIB 9518
*2. Pseudomonas aeruginosa* NCIB 8626
*3. Corynebacterium xerosis* NCIMB 9956
*4. Staphylococcus epidermidis NCTC 7291*
*5. Propionibacterium acnes* NCTC 737
*6. Pityrosporum ovale* ATCC 38593

The results are shown in Table 2:

**Table 2**

| Raw material | Concentration in agar (%) | Organism No. | | | | | |
|---|---|---|---|---|---|---|---|
| | | 1 | 2 | 3 | 4 | 5 | 6 |
| *Myrica gale* oil (Batch 03 042 001) | 3 | R | R | R | R | - | - |
| | 2.5 | R | R | R | R | - | R |
| | 2 | R | R | R | R | - | R |
| | 1.5 | R | R | R | R | - | R |
| | 1 | R | R | R | R | - | R |
| | 0.5 | + | + | + | + | - | + |
| | 0.25 | + | + | + | + | - | + |
| | 0.1 | + | + | + | + | + | + |

| | | | | | | | |
|---|---|---|---|---|---|---|---|
| Key: + = growth R = reduced growth - = no growth | | | | | | | |

The results indicate an MIC of 0.25% for the *Myrica gale* oil against *P. acnes* (acne-causing bacteria) and a MIC of 3% against *P. ovale* (yeast associated with dandruff).

### C. Antioxidant properties of Myrica gale

Lipid peroxidation (LPO) was measured using a commercially available colourimetric assay (K-assay) adapted for use with a 96 well microtitre plate reader. The plate was filled with linoleic acid dispersions, with and without Myrica gale, and one half was irradiated for 30 minutes using a solar simulator. LPO levels were compared with to a hydroperoxide standard and the screen was also repeated with Vitamin E as a positive comparison. The difference between the irradiated and non-irradiated samples was calculated to give the final percentage reduction in lipid peroxidation.

In these tests, *Myrica gale* was shown to have antioxidant properties comparable with those of Vitamin E and other antioxidant agents conventionally used in cosmetics and toiletries.

## Claims

1. *Myrica gale* oil, for use as an antioxidant agent in a topical skincare composition.

2. The use of *Myrica gale* oil as an antioxidant in a topical skincare composition.

3. The use of *Myrica gale* oil as an antioxidant agent in the manufacture of a topical skincare composition.

4. Oil or use as claimed in any one of Claims 1, 2 or 3, wherein the concentration of *Myrica gale* oil is in excess of 0.001 % by weight, more commonly in excess of 0.1 % by weight, and preferably in excess of 0.2% by weight of the composition.

5. Oil or use as claimed in any preceding claim, wherein the concentration of *Myrica gale* oil is preferably less than 50% by weight, more preferably less than 30% by weight, more preferably less than 10% by weight and most preferably less than 5% by weight of the composition.

6. Oil or use as claimed in any one of Claims 1, 2 or 3, wherein the concentration of *Myrica gale* oil falls in the range 0.001 % to 50% by weight of the composition, more preferably 0.1 % to 10% by weight and most preferably 0.2% to 5% by weight.

7. Oil or use as claimed in any preceding claim, wherein *Myrica gale* oil is obtained by steam distillation, water distillation, supercritical carbon dioxide extraction, supercritical water or subcritical (superheated) water extraction.

8. Oil or use as claimed in any preceding claim, wherein the composition comprises one or more excipients selected from the group consisting of humectants, emollients, surfactants, emulsifiers, chelating agents, sunscreening agents, preservatives, perfumes and colourings.

9. Oil or use as claimed in any preceding claim, wherein the composition comprises one or more additional active ingredients selected from the group consisting of herbal extracts, desquamatory actives, anti-acne actives, vitamin B3 compounds, retinoids, di-, tri-, tetra- and penta- peptides and derivatives thereof, hydroxy acids, antioxidants, chelators, anti-inflammatory agents, topical anaesthetics, tanning actives, skin lightening agents, anti-cellulite agents, flavenoids, antimicrobial actives, skin healing agents, antifungal actives, farnesol, phytantriol, allantoin, glucosamine and mixtures thereof.

10. Oil or use as claimed in Claim 9, wherein the composition comprises one or more additional antioxidant agents selected from the group consisting of sodium and magnesium ascorbyl phosphate, *Panax ginseng*, *Morus alba*, *Origanum vulgare* and *Rosmarinus officinalis* extracts, and mixtures thereof.

11. Oil or use as claimed in Claim 10 wherein the total amount of additional antioxidant agents ranges from 0.001 % to 10% by weight preferably 1 % to 7% by weight and more preferably 2% to 5% by weight of the composition.

12. Oil or use as claimed in Claim 9, wherein the composition comprises one or more herbal extracts selected from the group consisting of *Aesculus hippicastanum* (horsechestnut), *Equisetum arvense* (horse tail), *Arctium lappa* (burdock), *Ruscus aculeatus* (box holly), *Vitis vinifera* (grape), *Salix alba* (willowbark), *Betula pendula* (silver birch) extract, and mixtures thereof.

13. Oil or use as claimed in Claim 9, wherein the composition additionally comprises one or more anti-acne actives selected from the group consisting of salicylic acid, witch hazel, benzoyl peroxide, sulfur, sodium sulfacetamide, triclosan, glycolic acid, tea tree oil, rosemary, burdock, willow extract and mixtures thereof.

14. Oil or use as claimed in Claim 9, wherein the composition comprises one or more antimicrobial or antibacterial compounds selected from the group consisting of triclosan, neomycin, clindamycin, polymyxin, bacitracin, benzoyl peroxide, tetracylines such as doxycycline or minocycline, sulfa drugs such as sulfacetamide, penicillins, cephalosporins such as cephalexin, and quinolones such as lomefloxacin, olfoxacin, trovafloxacin, and mixtures thereof.

15. Oil or use as claimed in Claim 9, wherein the composition comprises one or more anti-inflammatory compounds selected from the group consisting of aloe vera gel, aloe vera, licorice extract, pilewort, Canadian willow root, zinc, and allantoin.
